# EUROPÄISCHE PATENTANMELDUNG

(11) **EP 1 847 599 A1**
(43) Veröffentlichungstag der Anmeldung: **24.10.2007**
(21) Anmeldenummer: 06003668.8
(22) Anmeldetag: 23.02.2006
(51) Int. Cl.: C12N 9/20, C12N 15/63, C12N 1/15, C12N 1/21, C11D 3/386, C12N 15/55

(54) **Polypeptide mit Perhydrolase Aktivität**

(71) Anmelder: Cognis IP Management GmbH, 49589 Düsseldorf (DE)
(72) Erfinder: Dubreucq, Eric, Prof. Dr., 34000 Montpellier (FR); Weiss, Albrecht, Dr., 40764 Langenfeld (DE); Moulin, Guy, Prof. Dr., 34980 Montferrier-sur-Lez (FR)
(74) Vertreter: Maurer, Stefanie

(57) **Zusammenfassung**

Vorgeschlagen werden Polypeptide mit Perhydrolase Aktivität mit einer Aminosäuresequenz, welche zu der in SEQ ID NO 3 angegebenen Aminosäuresequenz wenigstens 80 % Homologie oder wenigstens 65 % Identität besitzt, mit Ausnahme der SEQ ID NO 3.

Des weiteren werden Polypeptide mit Perhydrolase Aktivität vorgeschlagen, enthaltend wenigstens ein Motif, welches wenigstens 50 % Homologie oder wenigstens 70 % Identität zeigt zu einem Motif ausgewählt aus der Gruppe, die gebildet wird aus SEQ ID NO 4: GYSGGxxAxxWAxxxxxxYAPE, SEQ ID NO 5: GYSGGxxAxxWAxxxxxxYAPD, SEQ ID NO 6: GFSGGxxAxxWAxxxxxxYAPE, SEQ ID NO 7: GFSGGxxAxxWAxxxxxxYAPD, SEQ ID NO 8: GYSGGxxAxxWAxxxxxxYA und SEQ ID NO 9: GFSGGxxAxxWAxxxxxxYA.

SEQUENCE LISTING

<110> Cognis IP Management GmbH

<120> Polypeptide mit Perhydrolase Aktivität

<130> C 3090

<140> EP 06003668
<141> 2006-02-23

<160> 9

<170> PatentIn version 3.1

<210> 1
<211> 1398
<212> DNA
<213> Candida parapsilosis

<220>
<221> CDS
<222> (1)..(1398)
<223>

<400> 1
atg cgt tac ttt gct att gct ttc ttg ctc atc aat acc att tca gct 48
Met Arg Tyr Phe Ala Ile Ala Phe Leu Leu Ile Asn Thr Ile Ser Ala
1               5                   10                  15

ttt gtc ttg gct ccc aaa aag cca tct caa gac gat ttc tac act cca 96
Phe Val Leu Ala Pro Lys Lys Pro Ser Gln Asp Asp Phe Tyr Thr Pro
            20                  25                  30

cca caa ggt tat gaa gct caa cct ctt ggt tct att ttg aaa aca aga 144
Pro Gln Gly Tyr Glu Ala Gln Pro Leu Gly Ser Ile Leu Lys Thr Arg
        35                  40                  45

aac gtc ccc aat cca ttg act aat gtt ttc act cca gtt aaa gtt caa 192
Asn Val Pro Asn Pro Leu Thr Asn Val Phe Thr Pro Val Lys Val Gln
    50                  55                  60

aat gca tgg caa tta ttg gtt aga tct gaa gat aca ttt ggt aac cca 240

Asn Ala Trp Gln Leu Leu Val Arg Ser Glu Asp Thr Phe Gly Asn Pro
65                  70                  75                  80

aac gcc ata gtc act acc att att caa cct ttc aat gct aaa aag gat        288
Asn Ala Ile Val Thr Thr Ile Ile Gln Pro Phe Asn Ala Lys Lys Asp
                85                  90                  95

aag ctt gtt tct tat caa aca ttt gaa gat tct ggt aaa ttg gat tgt        336
Lys Leu Val Ser Tyr Gln Thr Phe Glu Asp Ser Gly Lys Leu Asp Cys
            100                 105                 110

gct cca tca tat gct att caa tat gga tcg gac att tcg act ttg acc        384
Ala Pro Ser Tyr Ala Ile Gln Tyr Gly Ser Asp Ile Ser Thr Leu Thr
        115                 120                 125

act caa ggt gaa atg tac tac atc tct gct tta tta gat caa ggt tac        432
Thr Gln Gly Glu Met Tyr Tyr Ile Ser Ala Leu Leu Asp Gln Gly Tyr
    130                 135                 140

tat gtt gtc act cct gat tac gag ggt cca aag agt aca ttc act gta        480
Tyr Val Val Thr Pro Asp Tyr Glu Gly Pro Lys Ser Thr Phe Thr Val
145                 150                 155                 160

ggg ttg caa tca gga aga gct act ttg aat tcg ctt aga gct act ttg        528
Gly Leu Gln Ser Gly Arg Ala Thr Leu Asn Ser Leu Arg Ala Thr Leu
                165                 170                 175

aaa tca gga aac ttg act ggt gtt tca tca gac gct gag aca tta ttg        576
Lys Ser Gly Asn Leu Thr Gly Val Ser Ser Asp Ala Glu Thr Leu Leu
            180                 185                 190

tgg ggt tat tca gga gga agt ctt gct tca gga tgg gct gct gct ata        624
Trp Gly Tyr Ser Gly Gly Ser Leu Ala Ser Gly Trp Ala Ala Ala Ile
        195                 200                 205

caa aaa gaa tat gct cca gag ttg agt aaa aac ttg ctt ggt gct gca        672
Gln Lys Glu Tyr Ala Pro Glu Leu Ser Lys Asn Leu Leu Gly Ala Ala
    210                 215                 220

ctt ggt gga ttc gtt aca aac att act gcc act gct gaa gct gtt gat        720
Leu Gly Gly Phe Val Thr Asn Ile Thr Ala Thr Ala Glu Ala Val Asp
225                 230                 235                 240

agt ggt cca ttt gca gga atc atc tcc aat gca ttg gct ggt att gga        768
Ser Gly Pro Phe Ala Gly Ile Ile Ser Asn Ala Leu Ala Gly Ile Gly
                245                 250                 255

aat gaa tac cct gat ttc aaa aac tat ctt ttg aaa aaa gtg tca cca        816
Asn Glu Tyr Pro Asp Phe Lys Asn Tyr Leu Leu Lys Lys Val Ser Pro
            260                 265                 270

ttg ctt tca atc act tat cgt ttg gga aac act cac tgt ttg ctt gat        864
Leu Leu Ser Ile Thr Tyr Arg Leu Gly Asn Thr His Cys Leu Leu Asp
        275                 280                 285

ggt ggt att gct tat ttc ggt aaa tca ttc ttt tcc aga att att aga        912
Gly Gly Ile Ala Tyr Phe Gly Lys Ser Phe Phe Ser Arg Ile Ile Arg
    290                 295                 300

tat ttc cct gat gga tgg gat ctt gtc aac caa gaa cct atc aaa acc        960
Tyr Phe Pro Asp Gly Trp Asp Leu Val Asn Gln Glu Pro Ile Lys Thr
305                 310                 315                 320

atc ttg caa gat aat gga ttg gtt tac caa cca aag gac ttg acc cca       1008
Ile Leu Gln Asp Asn Gly Leu Val Tyr Gln Pro Lys Asp Leu Thr Pro
                325                 330                 335

caa att cca tta ttc atc tac cac ggt acc ttg gat gca att gtc ccc       1056

Gln Ile Pro Leu Phe Ile Tyr His Gly Thr Leu Asp Ala Ile Val Pro
            340                 345                 350

att gtc aac tca aga aag aca ttc caa caa tgg tgt gat tgg gga ctc      1104
Ile Val Asn Ser Arg Lys Thr Phe Gln Gln Trp Cys Asp Trp Gly Leu
        355                 360                 365

aaa tct ggt gaa tat aat gaa gat ttg acc aat gga cac att act gaa      1152
Lys Ser Gly Glu Tyr Asn Glu Asp Leu Thr Asn Gly His Ile Thr Glu
    370                 375                 380

tca att gtg ggt gca cca gct gct ttg act tgg att atc aat cgt ttc      1200
Ser Ile Val Gly Ala Pro Ala Ala Leu Thr Trp Ile Ile Asn Arg Phe
385                 390                 395                 400

aat gga cag cct cca gtt gat gga tgt caa cat aat gtg aga gct tca      1248
Asn Gly Gln Pro Pro Val Asp Gly Cys Gln His Asn Val Arg Ala Ser
                405                 410                 415

aac ttg gaa tat cca gga act cca caa tca atc aag aat tac ttt gaa      1296
Asn Leu Glu Tyr Pro Gly Thr Pro Gln Ser Ile Lys Asn Tyr Phe Glu
            420                 425                 430

gct gca ttg cac gca att ttg ggc ttt gat ttg ggt cca gat gtt aag      1344
Ala Ala Leu His Ala Ile Leu Gly Phe Asp Leu Gly Pro Asp Val Lys
        435                 440                 445

aga gat aag gtt act ttg ggc gga ttg ctc aag ttg gaa cgt ttt gct      1392
Arg Asp Lys Val Thr Leu Gly Gly Leu Leu Lys Leu Glu Arg Phe Ala
    450                 455                 460

ttt tag                                                              1398
Phe
465

<210>  2

<211>  465

<212>  PRT

<213>  Candida parapsilosis

<400>  2

Met Arg Tyr Phe Ala Ile Ala Phe Leu Leu Ile Asn Thr Ile Ser Ala
1               5                   10                  15

Phe Val Leu Ala Pro Lys Lys Pro Ser Gln Asp Asp Phe Tyr Thr Pro
            20                  25                  30

Pro Gln Gly Tyr Glu Ala Gln Pro Leu Gly Ser Ile Leu Lys Thr Arg
        35                  40                  45

Asn Val Pro Asn Pro Leu Thr Asn Val Phe Thr Pro Val Lys Val Gln
    50                  55                  60

Asn Ala Trp Gln Leu Leu Val Arg Ser Glu Asp Thr Phe Gly Asn Pro
65                  70                  75                  80

Asn Ala Ile Val Thr Thr Ile Ile Gln Pro Phe Asn Ala Lys Lys Asp
                85                  90                  95

Lys Leu Val Ser Tyr Gln Thr Phe Glu Asp Ser Gly Lys Leu Asp Cys
            100                 105                 110

Ala Pro Ser Tyr Ala Ile Gln Tyr Gly Ser Asp Ile Ser Thr Leu Thr
        115                 120                 125

Thr Gln Gly Glu Met Tyr Tyr Ile Ser Ala Leu Leu Asp Gln Gly Tyr
    130                 135                 140

Tyr Val Val Thr Pro Asp Tyr Glu Gly Pro Lys Ser Thr Phe Thr Val
145                 150                 155                 160

Gly Leu Gln Ser Gly Arg Ala Thr Leu Asn Ser Leu Arg Ala Thr Leu
                165                 170                 175

Lys Ser Gly Asn Leu Thr Gly Val Ser Ser Asp Ala Glu Thr Leu Leu
            180                 185                 190

Trp Gly Tyr Ser Gly Gly Ser Leu Ala Ser Gly Trp Ala Ala Ala Ile
        195                 200                 205

Gln Lys Glu Tyr Ala Pro Glu Leu Ser Lys Asn Leu Leu Gly Ala Ala
    210                 215                 220

Leu Gly Gly Phe Val Thr Asn Ile Thr Ala Thr Ala Glu Ala Val Asp
225                 230                 235                 240

Ser Gly Pro Phe Ala Gly Ile Ile Ser Asn Ala Leu Ala Gly Ile Gly
                245                 250                 255

Asn Glu Tyr Pro Asp Phe Lys Asn Tyr Leu Leu Lys Lys Val Ser Pro
            260                 265                 270

Leu Leu Ser Ile Thr Tyr Arg Leu Gly Asn Thr His Cys Leu Leu Asp
        275                 280                 285

Gly Gly Ile Ala Tyr Phe Gly Lys Ser Phe Phe Ser Arg Ile Ile Arg
    290                 295                 300

Tyr Phe Pro Asp Gly Trp Asp Leu Val Asn Gln Glu Pro Ile Lys Thr
305                 310                 315                 320

Ile Leu Gln Asp Asn Gly Leu Val Tyr Gln Pro Lys Asp Leu Thr Pro
                325                 330                 335

Gln Ile Pro Leu Phe Ile Tyr His Gly Thr Leu Asp Ala Ile Val Pro
            340                 345                 350

Ile Val Asn Ser Arg Lys Thr Phe Gln Gln Trp Cys Asp Trp Gly Leu
        355                 360                 365

Lys Ser Gly Glu Tyr Asn Glu Asp Leu Thr Asn Gly His Ile Thr Glu
    370                 375                 380

Ser Ile Val Gly Ala Pro Ala Ala Leu Thr Trp Ile Ile Asn Arg Phe
385                 390                 395                 400

Asn Gly Gln Pro Pro Val Asp Gly Cys Gln His Asn Val Arg Ala Ser
                405                 410                 415

Asn Leu Glu Tyr Pro Gly Thr Pro Gln Ser Ile Lys Asn Tyr Phe Glu
            420                 425                 430

Ala Ala Leu His Ala Ile Leu Gly Phe Asp Leu Gly Pro Asp Val Lys
        435                 440                 445

Arg Asp Lys Val Thr Leu Gly Gly Leu Leu Lys Leu Glu Arg Phe Ala
    450                 455                 460

Phe
465

<210>  3

<211>  449

<212>  PRT

<213>  Candida parapsilosis

<400>  3

Phe Val Leu Ala Pro Lys Lys Pro Ser Gln Asp Asp Phe Tyr Thr Pro
1               5                   10                  15

Pro Gln Gly Tyr Glu Ala Gln Pro Leu Gly Ser Ile Leu Lys Thr Arg
            20                  25                  30

Asn Val Pro Asn Pro Leu Thr Asn Val Phe Thr Pro Val Lys Val Gln
        35                  40                  45

Asn Ala Trp Gln Leu Leu Val Arg Ser Glu Asp Thr Phe Gly Asn Pro
    50                  55                  60

Asn Ala Ile Val Thr Thr Ile Ile Gln Pro Phe Asn Ala Lys Lys Asp
65                  70                  75                  80

Lys Leu Val Ser Tyr Gln Thr Phe Glu Asp Ser Gly Lys Leu Asp Cys
                85                  90                  95

Ala Pro Ser Tyr Ala Ile Gln Tyr Gly Ser Asp Ile Ser Thr Leu Thr
            100                 105                 110

Thr Gln Gly Glu Met Tyr Tyr Ile Ser Ala Leu Leu Asp Gln Gly Tyr
        115                 120                 125

Tyr Val Val Thr Pro Asp Tyr Glu Gly Pro Lys Ser Thr Phe Thr Val
    130                 135                 140

Gly Leu Gln Ser Gly Arg Ala Thr Leu Asn Ser Leu Arg Ala Thr Leu
145                 150                 155                 160

Lys Ser Gly Asn Leu Thr Gly Val Ser Ser Asp Ala Glu Thr Leu Leu
                165                 170                 175

Trp Gly Tyr Ser Gly Gly Ser Leu Ala Ser Gly Trp Ala Ala Ala Ile
            180                 185                 190

Gln Lys Glu Tyr Ala Pro Glu Leu Ser Lys Asn Leu Leu Gly Ala Ala
        195                 200                 205

Leu Gly Gly Phe Val Thr Asn Ile Thr Ala Thr Ala Glu Ala Val Asp
    210                 215                 220

Ser Gly Pro Phe Ala Gly Ile Ile Ser Asn Ala Leu Ala Gly Ile Gly
225                 230                 235                 240

Asn Glu Tyr Pro Asp Phe Lys Asn Tyr Leu Leu Lys Lys Val Ser Pro
                245                 250                 255

Leu Leu Ser Ile Thr Tyr Arg Leu Gly Asn Thr His Cys Leu Leu Asp
            260                 265                 270

Gly Gly Ile Ala Tyr Phe Gly Lys Ser Phe Phe Ser Arg Ile Ile Arg
        275                 280                 285

Tyr Phe Pro Asp Gly Trp Asp Leu Val Asn Gln Glu Pro Ile Lys Thr
    290                 295                 300

Ile Leu Gln Asp Asn Gly Leu Val Tyr Gln Pro Lys Asp Leu Thr Pro
305                 310                 315                 320

Gln Ile Pro Leu Phe Ile Tyr His Gly Thr Leu Asp Ala Ile Val Pro
                325                 330                 335

Ile Val Asn Ser Arg Lys Thr Phe Gln Gln Trp Cys Asp Trp Gly Leu
            340                 345                 350

Lys Ser Gly Glu Tyr Asn Glu Asp Leu Thr Asn Gly His Ile Thr Glu
        355                 360                 365

Ser Ile Val Gly Ala Pro Ala Ala Leu Thr Trp Ile Ile Asn Arg Phe
    370                 375                 380

Asn Gly Gln Pro Pro Val Asp Gly Cys Gln His Asn Val Arg Ala Ser
385                 390                 395                 400

Asn Leu Glu Tyr Pro Gly Thr Pro Gln Ser Ile Lys Asn Tyr Phe Glu
                405                 410                 415

Ala Ala Leu His Ala Ile Leu Gly Phe Asp Leu Gly Pro Asp Val Lys
            420                 425                 430

Arg Asp Lys Val Thr Leu Gly Gly Leu Leu Lys Leu Glu Arg Phe Ala
        435                 440                 445

Phe

<210>  4
<211>  22
<212>  PRT
<213>  Artificial Sequence

<220>
<221>  MISC_FEATURE
<222>  (1)..(22)
<223>  /note="Description of artificial sequence: consensual motif"

<220>
<221>  MISC_FEATURE
<222>  (6)..(7)
<223>  Xaa is any essential or unessential amino acid

<220>
<221>  MISC_FEATURE
<222>  (9)..(10)
<223>  Xaa is any essential or unessential amino acid

<220>
<221>  MISC_FEATURE

<222>  (13)..(18)
<223>  Xaa is any essential or unessential amino acid

<400>  4

Gly Tyr Ser Gly Gly Xaa Xaa Ala Xaa Xaa Trp Ala Xaa Xaa Xaa Xaa
1               5                   10                  15

Xaa Xaa Tyr Ala Pro Glu
            20

<210>  5
<211>  22
<212>  PRT
<213>  Artificial Sequence

<220>
<221>  MISC_FEATURE
<222>  (1)..(22)
<223>  /note="Description of artificial sequence: consensual motif"

<220>
<221>  MISC_FEATURE
<222>  (6)..(7)
<223>  Xaa is any essential or nonessential amino acid

<220>
<221>  MISC_FEATURE
<222>  (9)..(10)
<223>  Xaa is any essential or nonessential amino acid

<220>
<221>  MISC_FEATURE
<222>  (13)..(18)
<223>  Xaa is any essential or nonessential amino acid

<400>  5

Gly Tyr Ser Gly Gly Xaa Xaa Ala Xaa Xaa Trp Ala Xaa Xaa Xaa Xaa
1               5                   10                  15

Xaa Xaa Tyr Ala Pro Asp
            20

<210>  6

<211>  22

<212>  PRT

<213>  Artificial Sequence

<220>

<221>  MISC_FEATURE

<222>  (1)..(22)

<223>  /note="Description of artificial sequence: consensual motif"

<220>

<221>  MISC_FEATURE

<222>  (6)..(7)

<223>  Xaa is any essential or nonessential amino acid

<220>

<221>  MISC_FEATURE

<222>  (9)..(10)

<223>  Xaa is any essential or nonessential amino acid

<220>

<221>  MISC_FEATURE

<222>  (13)..(18)

<223>  Xaa is any essential or nonessential amino acid

<400>  6

Gly Phe Ser Gly Gly Xaa Xaa Ala Xaa Xaa Trp Ala Xaa Xaa Xaa Xaa
1               5                   10                  15

Xaa Xaa Tyr Ala Pro Glu
            20

<210>  7

<211>  22

<212>  PRT

<213>  Artificial Sequence

<220>

<221>  MISC_FEATURE

<222>  (1)..(22)

<223>  /note="Description of artificial sequence: consensual motif"

<220>

<221>  MISC_FEATURE

<222>  (6)..(7)

<223>  Xaa is any essential or nonessential amino acid

<220>

<221>  MISC_FEATURE

<222>  (9)..(10)

<223>  Xaa is any essential or nonessential amino acid

<220>

<221>  MISC_FEATURE

<222>  (13)..(18)

<223>  Xaa is any essential or nonessential amino acid

<400>  7

Gly Phe Ser Gly Gly Xaa Xaa Ala Xaa Xaa Trp Ala Xaa Xaa Xaa Xaa
1               5                   10                  15

Xaa Xaa Tyr Ala Pro Asp
            20

<210>  8

<211>  20

<212>  PRT

<213>  Artificial Sequence

<220>

<221> MISC_FEATURE

<222> (1)..(20)

<223> /note="Description of artificial sequence: consensual motif"

<220>

<221> MISC_FEATURE

<222> (6)..(7)

<223> Xaa is any essential or nonessential amino acid

<220>

<221> MISC_FEATURE

<222> (9)..(10)

<223> Xaa is any essential or nonessential amino acid

<220>

<221> MISC_FEATURE

<222> (13)..(18)

<223> Xaa is any essential or nonessential amino acid

<400> 8

Gly Tyr Ser Gly Gly Xaa Xaa Ala Xaa Xaa Trp Ala Xaa Xaa Xaa Xaa
1               5                   10                  15

Xaa Xaa Tyr Ala
            20

<210> 9

<211> 20

<212> PRT

<213> Artificial Sequence

<220>

<221> MISC_FEATURE

<222> (1)..(20)

<223>  /note="Description of artificial sequence: consensual motif"

<220>
<221>  MISC_FEATURE
<222>  (6)..(7)
<223>  Xaa is any essential or nonessential amino acid

<220>
<221>  MISC_FEATURE
<222>  (9)..(10)
<223>  Xaa is any essential or nonessential amino acid

<220>
<221>  MISC_FEATURE
<222>  (13)..(18)
<223>  Xaa is any essential or nonessential amino acid

<400>  9
Gly Phe Ser Gly Gly Xaa Xaa Ala Xaa Xaa Trp Ala Xaa Xaa Xaa Xaa
1               5                   10                  15

Xaa Xaa Tyr Ala
            20

SEQUENCE LISTING

<110>  Cognis IP Management GmbH

<120>  Polypeptide mit Perhydrolase Aktivität

<130>  C 3090

<160>  9

<170>  PatentIn version 3.1

<210>  1
<211>  1398
<212>  DNA
<213>  Candida parapsilosis

<220>
<221>  CDS
<222>  (1)..(1398)
<223>

<400>  1
atg cgt tac ttt gct att gct ttc ttg ctc atc aat acc att tca gct      48
Met Arg Tyr Phe Ala Ile Ala Phe Leu Leu Ile Asn Thr Ile Ser Ala
1               5                   10                  15

ttt gtc ttg gct ccc aaa aag cca tct caa gac gat ttc tac act cca      96
Phe Val Leu Ala Pro Lys Lys Pro Ser Gln Asp Asp Phe Tyr Thr Pro
            20                  25                  30

cca caa ggt tat gaa gct caa cct ctt ggt tct att ttg aaa aca aga      144
Pro Gln Gly Tyr Glu Ala Gln Pro Leu Gly Ser Ile Leu Lys Thr Arg
        35                  40                  45

aac gtc ccc aat cca ttg act aat gtt ttc act cca gtt aaa gtt caa      192
Asn Val Pro Asn Pro Leu Thr Asn Val Phe Thr Pro Val Lys Val Gln
    50                  55                  60

aat gca tgg caa tta ttg gtt aga tct gaa gat aca ttt ggt aac cca      240
Asn Ala Trp Gln Leu Leu Val Arg Ser Glu Asp Thr Phe Gly Asn Pro
65                  70                  75                  80

aac gcc ata gtc act acc att att caa cct ttc aat gct aaa aag gat      288
Asn Ala Ile Val Thr Thr Ile Ile Gln Pro Phe Asn Ala Lys Lys Asp
                85                  90                  95

aag ctt gtt tct tat caa aca ttt gaa gat tct ggt aaa ttg gat tgt 336
Lys Leu Val Ser Tyr Gln Thr Phe Glu Asp Ser Gly Lys Leu Asp Cys
            100                 105                 110

gct cca tca tat gct att caa tat gga tcg gac att tcg act ttg acc 384
Ala Pro Ser Tyr Ala Ile Gln Tyr Gly Ser Asp Ile Ser Thr Leu Thr
        115                 120                 125

act caa ggt gaa atg tac tac atc tct gct tta tta gat caa ggt tac 432
Thr Gln Gly Glu Met Tyr Tyr Ile Ser Ala Leu Leu Asp Gln Gly Tyr
    130                 135                 140

tat gtt gtc act cct gat tac gag ggt cca aag agt aca ttc act gta 480
Tyr Val Val Thr Pro Asp Tyr Glu Gly Pro Lys Ser Thr Phe Thr Val
145                 150                 155                 160

ggg ttg caa tca gga aga gct act ttg aat tcg ctt aga gct act ttg 528
Gly Leu Gln Ser Gly Arg Ala Thr Leu Asn Ser Leu Arg Ala Thr Leu
                165                 170                 175

aaa tca gga aac ttg act ggt gtt tca tca gac gct gag aca tta ttg 576
Lys Ser Gly Asn Leu Thr Gly Val Ser Ser Asp Ala Glu Thr Leu Leu
            180                 185                 190

tgg ggt tat tca gga gga agt ctt gct tca gga tgg gct gct gct ata 624
Trp Gly Tyr Ser Gly Gly Ser Leu Ala Ser Gly Trp Ala Ala Ala Ile
        195                 200                 205

caa aaa gaa tat gct cca gag ttg agt aaa aac ttg ctt ggt gct gca 672
Gln Lys Glu Tyr Ala Pro Glu Leu Ser Lys Asn Leu Leu Gly Ala Ala
    210                 215                 220

ctt ggt gga ttc gtt aca aac att act gcc act gct gaa gct gtt gat 720
Leu Gly Gly Phe Val Thr Asn Ile Thr Ala Thr Ala Glu Ala Val Asp
225                 230                 235                 240

agt ggt cca ttt gca gga atc atc tcc aat gca ttg gct ggt att gga 768
Ser Gly Pro Phe Ala Gly Ile Ile Ser Asn Ala Leu Ala Gly Ile Gly
                245                 250                 255

aat gaa tac cct gat ttc aaa aac tat ctt ttg aaa aaa gtg tca cca 816
Asn Glu Tyr Pro Asp Phe Lys Asn Tyr Leu Leu Lys Lys Val Ser Pro
            260                 265                 270

ttg ctt tca atc act tat cgt ttg gga aac act cac tgt ttg ctt gat 864
Leu Leu Ser Ile Thr Tyr Arg Leu Gly Asn Thr His Cys Leu Leu Asp
        275                 280                 285

ggt ggt att gct tat ttc ggt aaa tca ttc ttt tcc aga att att aga 912
Gly Gly Ile Ala Tyr Phe Gly Lys Ser Phe Phe Ser Arg Ile Ile Arg
    290                 295                 300

tat ttc cct gat gga tgg gat ctt gtc aac caa gaa cct atc aaa acc 960
Tyr Phe Pro Asp Gly Trp Asp Leu Val Asn Gln Glu Pro Ile Lys Thr
305                 310                 315                 320

atc ttg caa gat aat gga ttg gtt tac caa cca aag gac ttg acc cca 1008
Ile Leu Gln Asp Asn Gly Leu Val Tyr Gln Pro Lys Asp Leu Thr Pro
                325                 330                 335

caa att cca tta ttc atc tac cac ggt acc ttg gat gca att gtc ccc 1056
Gln Ile Pro Leu Phe Ile Tyr His Gly Thr Leu Asp Ala Ile Val Pro
            340                 345                 350

att gtc aac tca aga aag aca ttc caa caa tgg tgt gat tgg gga ctc 1104
Ile Val Asn Ser Arg Lys Thr Phe Gln Gln Trp Cys Asp Trp Gly Leu
        355                 360                 365

aaa tct ggt gaa tat aat gaa gat ttg acc aat gga cac att act gaa     1152
Lys Ser Gly Glu Tyr Asn Glu Asp Leu Thr Asn Gly His Ile Thr Glu
    370                 375                 380

tca att gtg ggt gca cca gct gct ttg act tgg att atc aat cgt ttc     1200
Ser Ile Val Gly Ala Pro Ala Ala Leu Thr Trp Ile Ile Asn Arg Phe
385                 390                 395                 400

aat gga cag cct cca gtt gat gga tgt caa cat aat gtg aga gct tca     1248
Asn Gly Gln Pro Pro Val Asp Gly Cys Gln His Asn Val Arg Ala Ser
                405                 410                 415

aac ttg gaa tat cca gga act cca caa tca atc aag aat tac ttt gaa     1296
Asn Leu Glu Tyr Pro Gly Thr Pro Gln Ser Ile Lys Asn Tyr Phe Glu
            420                 425                 430

gct gca ttg cac gca att ttg ggc ttt gat ttg ggt cca gat gtt aag     1344
Ala Ala Leu His Ala Ile Leu Gly Phe Asp Leu Gly Pro Asp Val Lys
        435                 440                 445

aga gat aag gtt act ttg ggc gga ttg ctc aag ttg gaa cgt ttt gct     1392
Arg Asp Lys Val Thr Leu Gly Gly Leu Leu Lys Leu Glu Arg Phe Ala
    450                 455                 460

ttt tag                                                             1398
Phe
465

<210>  2

<211>  465

<212>  PRT

<213>  Candida parapsilosis

<400>  2

Met Arg Tyr Phe Ala Ile Ala Phe Leu Leu Ile Asn Thr Ile Ser Ala
1               5                   10                  15

Phe Val Leu Ala Pro Lys Lys Pro Ser Gln Asp Asp Phe Tyr Thr Pro
            20                  25                  30

Pro Gln Gly Tyr Glu Ala Gln Pro Leu Gly Ser Ile Leu Lys Thr Arg
        35                  40                  45

Asn Val Pro Asn Pro Leu Thr Asn Val Phe Thr Pro Val Lys Val Gln
    50                  55                  60

Asn Ala Trp Gln Leu Leu Val Arg Ser Glu Asp Thr Phe Gly Asn Pro
65                  70                  75                  80

Asn Ala Ile Val Thr Thr Ile Ile Gln Pro Phe Asn Ala Lys Lys Asp
                85                  90                  95

Lys Leu Val Ser Tyr Gln Thr Phe Glu Asp Ser Gly Lys Leu Asp Cys
            100                 105                 110

Ala Pro Ser Tyr Ala Ile Gln Tyr Gly Ser Asp Ile Ser Thr Leu Thr
        115                 120                 125

Thr Gln Gly Glu Met Tyr Tyr Ile Ser Ala Leu Leu Asp Gln Gly Tyr
    130                 135                 140

Tyr Val Val Thr Pro Asp Tyr Glu Gly Pro Lys Ser Thr Phe Thr Val
145                 150                 155                 160

Gly Leu Gln Ser Gly Arg Ala Thr Leu Asn Ser Leu Arg Ala Thr Leu
                165                 170                 175

Lys Ser Gly Asn Leu Thr Gly Val Ser Ser Asp Ala Glu Thr Leu Leu
            180                 185                 190

Trp Gly Tyr Ser Gly Gly Ser Leu Ala Ser Gly Trp Ala Ala Ile
        195                 200                 205

Gln Lys Glu Tyr Ala Pro Glu Leu Ser Lys Asn Leu Leu Gly Ala Ala
    210                 215                 220

Leu Gly Gly Phe Val Thr Asn Ile Thr Ala Thr Ala Glu Ala Val Asp
225                 230                 235                 240

Ser Gly Pro Phe Ala Gly Ile Ile Ser Asn Ala Leu Ala Gly Ile Gly
                245                 250                 255

Asn Glu Tyr Pro Asp Phe Lys Asn Tyr Leu Leu Lys Lys Val Ser Pro
            260                 265                 270

Leu Leu Ser Ile Thr Tyr Arg Leu Gly Asn Thr His Cys Leu Leu Asp
        275                 280                 285

Gly Gly Ile Ala Tyr Phe Gly Lys Ser Phe Phe Ser Arg Ile Ile Arg
    290                 295                 300

Tyr Phe Pro Asp Gly Trp Asp Leu Val Asn Gln Glu Pro Ile Lys Thr
305                 310                 315                 320

Ile Leu Gln Asp Asn Gly Leu Val Tyr Gln Pro Lys Asp Leu Thr Pro
                325                 330                 335

Gln Ile Pro Leu Phe Ile Tyr His Gly Thr Leu Asp Ala Ile Val Pro
            340                 345                 350

Ile Val Asn Ser Arg Lys Thr Phe Gln Gln Trp Cys Asp Trp Gly Leu
        355                 360                 365

Lys Ser Gly Glu Tyr Asn Glu Asp Leu Thr Asn Gly His Ile Thr Glu
    370                 375                 380

Ser Ile Val Gly Ala Pro Ala Ala Leu Thr Trp Ile Ile Asn Arg Phe
385                 390                 395                 400

Asn Gly Gln Pro Pro Val Asp Gly Cys Gln His Asn Val Arg Ala Ser
                405                 410                 415

Asn Leu Glu Tyr Pro Gly Thr Pro Gln Ser Ile Lys Asn Tyr Phe Glu
            420                 425                 430

Ala Ala Leu His Ala Ile Leu Gly Phe Asp Leu Gly Pro Asp Val Lys
        435                 440                 445

Arg Asp Lys Val Thr Leu Gly Gly Leu Leu Lys Leu Glu Arg Phe Ala
    450                 455                 460

Phe
465

<210>  3

<211>  449

<212>  PRT

<213>  Candida parapsilosis


<400>  3

Phe Val Leu Ala Pro Lys Lys Pro Ser Gln Asp Asp Phe Tyr Thr Pro
1               5                   10                  15

Pro Gln Gly Tyr Glu Ala Gln Pro Leu Gly Ser Ile Leu Lys Thr Arg
            20                  25                  30

Asn Val Pro Asn Pro Leu Thr Asn Val Phe Thr Pro Val Lys Val Gln
        35                  40                  45

Asn Ala Trp Gln Leu Leu Val Arg Ser Glu Asp Thr Phe Gly Asn Pro
    50                  55                  60

Asn Ala Ile Val Thr Thr Ile Ile Gln Pro Phe Asn Ala Lys Lys Asp
65                  70                  75                  80

Lys Leu Val Ser Tyr Gln Thr Phe Glu Asp Ser Gly Lys Leu Asp Cys
                85                  90                  95

Ala Pro Ser Tyr Ala Ile Gln Tyr Gly Ser Asp Ile Ser Thr Leu Thr
            100                 105                 110

Thr Gln Gly Glu Met Tyr Tyr Ile Ser Ala Leu Leu Asp Gln Gly Tyr
        115                 120                 125

Tyr Val Val Thr Pro Asp Tyr Glu Gly Pro Lys Ser Thr Phe Thr Val
    130                 135                 140

Gly Leu Gln Ser Gly Arg Ala Thr Leu Asn Ser Leu Arg Ala Thr Leu
145                 150                 155                 160

Lys Ser Gly Asn Leu Thr Gly Val Ser Ser Asp Ala Glu Thr Leu Leu
                165                 170                 175

Trp Gly Tyr Ser Gly Gly Ser Leu Ala Ser Gly Trp Ala Ala Ala Ile
            180                 185                 190

Gln Lys Glu Tyr Ala Pro Glu Leu Ser Lys Asn Leu Leu Gly Ala Ala
        195                 200                 205

Leu Gly Gly Phe Val Thr Asn Ile Thr Ala Thr Ala Glu Ala Val Asp
    210                 215                 220

Ser Gly Pro Phe Ala Gly Ile Ile Ser Asn Ala Leu Ala Gly Ile Gly
225                 230                 235                 240

Asn Glu Tyr Pro Asp Phe Lys Asn Tyr Leu Leu Lys Lys Val Ser Pro
                245                 250                 255

Leu Leu Ser Ile Thr Tyr Arg Leu Gly Asn Thr His Cys Leu Leu Asp
            260                 265                 270

Gly Gly Ile Ala Tyr Phe Gly Lys Ser Phe Phe Ser Arg Ile Ile Arg
        275                 280                 285

Tyr Phe Pro Asp Gly Trp Asp Leu Val Asn Gln Glu Pro Ile Lys Thr
    290                 295                 300

Ile Leu Gln Asp Asn Gly Leu Val Tyr Gln Pro Lys Asp Leu Thr Pro
305                 310                 315                 320

Gln Ile Pro Leu Phe Ile Tyr His Gly Thr Leu Asp Ala Ile Val Pro
                325                 330                 335

Ile Val Asn Ser Arg Lys Thr Phe Gln Gln Trp Cys Asp Trp Gly Leu
            340                 345                 350

Lys Ser Gly Glu Tyr Asn Glu Asp Leu Thr Asn Gly His Ile Thr Glu
        355                 360                 365

Ser Ile Val Gly Ala Pro Ala Ala Leu Thr Trp Ile Ile Asn Arg Phe
    370                 375                 380

Asn Gly Gln Pro Pro Val Asp Gly Cys Gln His Asn Val Arg Ala Ser
385                 390                 395                 400

Asn Leu Glu Tyr Pro Gly Thr Pro Gln Ser Ile Lys Asn Tyr Phe Glu
                405                 410                 415

Ala Ala Leu His Ala Ile Leu Gly Phe Asp Leu Gly Pro Asp Val Lys
            420                 425                 430

Arg Asp Lys Val Thr Leu Gly Gly Leu Leu Lys Leu Glu Arg Phe Ala
        435                 440                 445

Phe

<210>  4
<211>  22
<212>  PRT
<213>  artificial

<220>
<221>  MISC_FEATURE
<222>  (6)..(7)
<223>  Xaa is any essential or unessential amino acid

<220>
<221>  MISC_FEATURE
<222>  (9)..(10)
<223>  Xaa is any essential or unessential amino acid

<220>
<221>  MISC_FEATURE
<222>  (13)..(18)
<223>  Xaa is any essential or unessential amino acid

<400>  4
Gly Tyr Ser Gly Gly Xaa Xaa Ala Xaa Xaa Trp Ala Xaa Xaa Xaa Xaa
1               5                   10                  15

Xaa Xaa Tyr Ala Pro Glu
            20

<210>  5

<211>  22

<212>  PRT

<213>  artificial

<220>

<221>  MISC_FEATURE

<222>  (6)..(7)

<223>  Xaa is any essential or nonessential amino acid

<220>

<221>  MISC_FEATURE

<222>  (9)..(10)

<223>  Xaa is any essential or nonessential amino acid

<220>

<221>  MISC_FEATURE

<222>  (13)..(18)

<223>  Xaa is any essential or nonessential amino acid

<400>  5

Gly Tyr Ser Gly Gly Xaa Xaa Ala Xaa Xaa Trp Ala Xaa Xaa Xaa Xaa
1               5                   10                  15

Xaa Xaa Tyr Ala Pro Asp
            20

<210>  6

<211>  22

<212>  PRT

<213>  artificial

<220>

<221>  MISC_FEATURE

<222>  (6)..(7)

<223>  Xaa is any essential or nonessential amino acid

<220>

<221>  MISC_FEATURE

<222>  (9)..(10)

<223>  Xaa is any essential or nonessential amino acid


<220>

<221>  MISC_FEATURE

<222>  (13)..(18)

<223>  Xaa is any essential or nonessential amino acid


<400>  6

Gly Phe Ser Gly Gly Xaa Xaa Ala Xaa Xaa Trp Ala Xaa Xaa Xaa Xaa
1               5                   10                  15

Xaa Xaa Tyr Ala Pro Glu
            20

<210>  7

<211>  22

<212>  PRT

<213>  artificial


<220>

<221>  MISC_FEATURE

<222>  (6)..(7)

<223>  Xaa is any essential or nonessential amino acid


<220>

<221>  MISC_FEATURE

<222>  (9)..(10)

<223>  Xaa is any essential or nonessential amino acid


<220>

<221>  MISC_FEATURE

<222>  (13)..(18)

<223>  Xaa is any essential or nonessential amino acid

<400>  7

Gly Phe Ser Gly Gly Xaa Xaa Ala Xaa Xaa Trp Ala Xaa Xaa Xaa Xaa
1               5                   10                  15

Xaa Xaa Tyr Ala Pro Asp
            20

<210>  8

<211>  20

<212>  PRT

<213>  artificial

<220>

<221>  MISC_FEATURE

<222>  (6)..(7)

<223>  Xaa is any essential or nonessential amino acid

<220>

<221>  MISC_FEATURE

<222>  (9)..(10)

<223>  Xaa is any essential or nonessential amino acid

<220>

<221>  MISC_FEATURE

<222>  (13)..(18)

<223>  Xaa is any essential or nonessential amino acid

<400>  8

Gly Tyr Ser Gly Gly Xaa Xaa Ala Xaa Xaa Trp Ala Xaa Xaa Xaa Xaa
1               5                   10                  15

Xaa Xaa Tyr Ala
            20

<210>  9

<211>  20

<212>  PRT

<213>  artificial

<220>

<221>  MISC_FEATURE

<222>  (6)..(7)

<223>  Xaa is any essential or nonessential amino acid

<220>

<221>  MISC_FEATURE

<222>  (9)..(10)

<223>  Xaa is any essential or nonessential amino acid

<220>

<221>  MISC_FEATURE

<222>  (13)..(18)

<223>  Xaa is any essential or nonessential amino acid

<400>  9

Gly Phe Ser Gly Gly Xaa Xaa Ala Xaa Xaa Trp Ala Xaa Xaa Xaa Xaa
1               5                   10                  15

Xaa Xaa Tyr Ala
            20

## Beschreibung

### Gebiet der Erfindung

Die vorliegende Erfindung betrifft Polypeptide mit Perhydrolase Aktivität, Verfahren zur ihrer Gewinnung sowie Mittel zur Pflege, Reinigung, Bleiche oder Desinfektion enthaltend diese Polypeptide mit Perhydrolase Aktivität sowie deren Verwendung. Des Weiteren betrifft die Erfindung die für diese Polypeptide codierenden Nucleinsäuren.

### Stand der Technik

In der chemischen und biochemischen Synthese werden vermehrt Enzyme als Katalysatoren eingesetzt. So werden in vielen Fällen aufgrund der oft milderen Reaktionsbedingungen bereits in großtechnischen Verfahren Esterasen, speziell Lipasen (EC 3.1.1.3) zur Fettspaltung, Veresterung und Umesterung eingesetzt.
In Wasch- oder Reinigungsmitteln werden Enzyme seit langem eingesetzt um Reinigungseffekte zu erzielen oder zu verbessern. Zur Anwendung kommen v.a. Proteasen, Amylasen oder Esterasen, speziell Lipasen.

Für die herkömmliche chemische Bleiche von Wäsche werden anorganische, hoch alkalische Wasserstoffperoxidlieferanten wie Percarbonat oder Perborat in Kombination mit Bleichboostem (TAED oder NOBS) eingesetzt. Dieses Standard-Bleichsystem ist aber nur bei Temperaturen ab 50° bis 60°C voll wirksam. Für den Niedrigtemperaturbereich steht kein optimales Bleichsystem zur Verfügung. Außerdem kann es zu lokalen "spotting"-Effekten bei gefärbten Textilien kommen. Vor allem kann aber eine lokale Überkonzentration der Bleichbooster beim Auflösen in unmittelbarer Nähe von Textilien zu Oxidationsschäden führen. Die Bleichkomponente Wasserstoffperoxid entsteht durch spontanen Zerfall des Salzes und führt damit schnell aber kurzfristig zu hohen Konzentrationen. Eine schonende, verzögerte Bleiche bei milderem pH Wert ist nicht möglich.
Für den Einsatz in schwach basischen Matrizes z.B. Flüssigformulierungen steht ebenfalls kein optimales Bleichsystem zur Verfügung.

Längerkettige Persäuren sind geeignet, auch bei Temperaturen zwischen 30°- 40° C gut zu bleichen. Persäuren zur Bleiche können aufgrund der Instabilität nicht direkt in die Waschmittelformulierung eingearbeitet werden, sie müssen *in situ* hergestellt werden.

Hierzu werden die oben genannten Bleichbooster verwendet. TAED setzt beispielsweise in Kombination mit Wasserstoffperoxid Peressigsäure frei.

Die enzymatische Herstellung von Persäuren ist seit langem bekannt. Der Einsatz dieser Persäuren wird beschrieben zur Bleiche, Desinfektion, in Waschmitteln, Reinigungsmitteln oder ähnlichem. Es werden dabei Enzyme verwendet, die in der Lage sind, Perhydrolyse-Reaktionen durchzuführen ausgehend von Estern. Die technischen Enzyme, die hierfür in Frage kommen, sind Lipasen, Perhydrolasen, Acyltransferasen, Esterasen und andere Hydrolasen. Diese Enzyme zeigen neben der gewünschten Perhydrolyse-Reaktion auch ihre natürlichen Hydrolysereaktionen. Dies führt dazu, dass der eingesetzte Ester für unerwünschte Reaktionen verbraucht wird und ein sehr geringes Verhältnis von Perhydrolyse zu Hydrolyse beobachtet wird. Dies führt dazu, dass die gewünschte Bildung von Persäuren unterdrückt wird zu Gunsten der Bildung von Säuren. Dies hat den Effekt, dass eine größere Konzentration an Ester eingesetzt werden muss um eine ausreichende Persäurekonzentration zu erreichen. Dies wiederum führt zu hohen Kosten, zu Formulierungsproblemen und weiteren Nachteilen.

Die beschriebenen Enzymsysteme führen zur Bildung von Persäuren. Normalerweise haben diese Enzyme auch die Eigenschaft, Persäuren zu den entsprechenden Säuren zu hydrolysieren, also eine Rückreaktion des gebildeten Produkts zu katalysieren. So können solche Enzyme gezielt zur Reduktion oder Zerstörung von Persäuren eingesetzt werden. In den beschriebenen Anwendungsfällen ist man jedoch an einer relativ hohen Persäurekonzentration interessiert und damit an einer langsamer ablaufenden Hydrolyse der Persäuren. Dass dies so ist, zeigt die Existenz der Persäure im Anwendungssystem.

Diese Enzyme, die zur Bildung der Persäuren verwendet wurden, werden direkt in Waschmittel oder Reinigungsmittel zugemischt.
In der WO2005/056782 wird eine Perhydrolase aus *M. smegmatis* beschrieben, die in Waschmitteln oder anderen Reinigungs- oder Bleichsystemen Verwendung findet.

Es besteht weiterhin Bedarf an Enzymsystemen für die beschriebenen Anwendungen, für verbesserte Methoden zur Entwicklung solcher Systeme und an verbesserten Reinigungs-, Desinfizierungs- Oxidations- oder Bleichmittel. Diese Mittel sollen kostengünstig, effektiv vor allem bei niedrigen Temperaturen, mild und leicht in der Handhabung sein.

Es lag daher die Aufgabe zugrunde, ein Enzymsystem zu finden, welches eine hohe Affinität für Wasserstoffperoxid im Vergleich zu Wasser aufweist, damit die Hydrolyse im Vergleich zur Perhydrolyse eine Nebenreaktion wird und die Enzym-/Wasserstoffperoxid/Estersysteme effektiver eingesetzt werden können und es nur eine beschränkte Katalyse der Persäure-Rückreaktion gibt.

### Beschreibung, der Erfindung

Gegenstand der Erfindung sind Polypeptide mit Perhydrolase Aktivität mit einer Aminosäuresequenz, welche zu der in SEQ ID NO 3 angegebenen Aminosäuresequenz wenigstens 80 % Homologie besitzen, mit Ausnahme der SEQ ID NO 3.
Besonders bevorzugt sind Polypeptide mit Perhydrolase Aktivität, deren Aminosäuresequenz mit einer der in SEQ ID NO 3 angegebenen Aminosäuresequenz zu mindestens 83 %, vorzugsweise mindestens 85 %, insbesondere mindestens 90 %, besonders bevorzugt mindestens 95 %, 96 %, 96,5 %, 97 %, 97,5 %, 98 %, 98,5 %, 99 % und ganz besonders bevorzugt 99,5% Homologie besitzen.

Ein weiterer Gegenstand der Erfindung sind Polypeptide mit Perhydrolase Aktivität mit einer Aminosäuresequenz, welche zu der in SEQ ID NO 3 angegebenen Aminosäuresequenz wenigstens 65% Identität besitzt, mit Ausnahme der SEQ ID NO 3.
Besonders bevorzugt sind Polypeptide mit Perhydrolase Aktivität deren Aminosäuresequenz mit einer der in SEQ ID NO 3 angegebenen Aminosäuresequenz zu mindestens 70 %, mindestens 75 %, mindestens 80 %, vorzugsweise mindestens 85 %, insbesondere mindestens 90 %, besonders bevorzugt mindestens 95 %, 96 %, 96,5 %, 97 %, 97,5 %, 98 %, 98,5 %, 99 % und ganz besonders bevorzugt 99,5 % Identität besitzt.

In Sinne der Erfindung wird unter **Homologie** in Bezug auf die Aminosäuresequenz Variationen an einzelnen Aminosäuren in der Sequenz verstanden, die die Funktion des Polypeptides nicht verändern. Ein Polypeptid mit mindestens 80 % Homologie besitzt also in maximal 19,9 % der Positionen equivalent zur SEQ ID NO 3 einen variablen Aminosäureaustausch und an 80 % der Positionen equivalent zur SEQ ID NO 3 in der Aminosäuresequenz entweder identische Aminosäuren oder Aminosäuren, die durch gleichwertige Funktion, gleiche Ladung oder gleiche Hydrophobie besetzt sind und so ausgewählt sind, dass das resultierende Polypeptid eine gleichwertige oder verbesserte Perhydrolyse Aktivität zeigt. Diese Homologiebestimmung erfolgt durch Strukturvergleich mit alignments mit Hilfe von Computerprogrammen und speziellen Algorithmen wie bevorzugt BLAST, CLUSTAL X, ALIGN, PASTA, FASTA, PILEUP, BESTFIT, GAP. Erfindungsgemäß wird das alignment bevorzugt durch den BLAST Algorithmus mit der BLOSUM 62 Matrix durchgeführt.

Unter dem Begriff "**Identität**" in Bezug auf die Aminosäuresequenz wird eine exakte Gleichheit der Aminosäuren an den Positionen equivalent zu der Bezugsaminosäuresequenz verstanden. Für ein Polypeptide mit Perhydrolase Aktivität mit einer Aminosäuresequenz, welche zu der in SEQ ID NO 3 angegebenen Aminosäuresequenz wenigstens 65% Identität besitzt bedeutet das, dass wenigstens 65 % der Positionen in einer Aminosäuresequenz äquivalent zu den Positionen in der SEQ ID NO 3 exakt die gleiche Aminosäure besitzen.

Eine Position in einer Aminosäuresequenz wird dann als äquivalent zu einer Position in einer anderen Aminosäuresequenz verstanden, wenn diese Positionen nach dem Sequenzvergleich über "alignment" übereinstimmen, sich also entsprechen.

Die Methoden des alignments sind dem Fachmann bekannt und werden durchgeführt mit Computerprogrammen und speziellen Algorithmen wie bevorzugt BLAST, CLUSTAL X, ALIGN, PASTA, FASTA, PILEUP, BESTFIT, GAP. Erfindungsgemäß wird das alignment bevorzugt durch den BLAST Algorithmus mit der BLOSUM 62 Matrix durchgeführt.

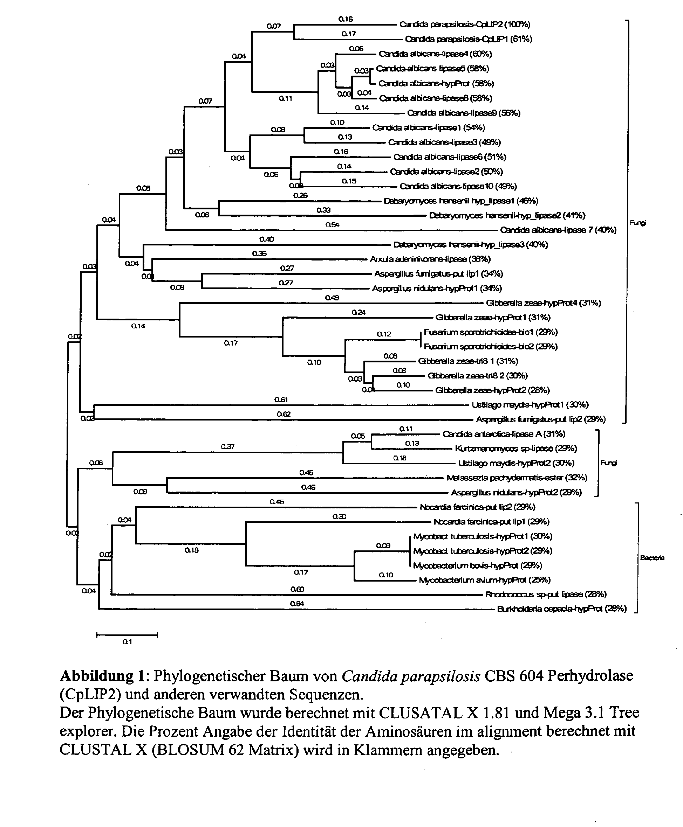

Mit diesen Programmen ist es teilweise auch möglich, so genannte **Phylogenetische Bäume** zu erstellen, die den Verwandtschaftsgrad bestimmter Enzyme zu anderen Enzymen bzw. Polypeptiden und deren Identitäten darstellen können.. Der Phylogenetische Baum zur SEQ ID NO 3 ist in **Abbildung 1** dargestellt.

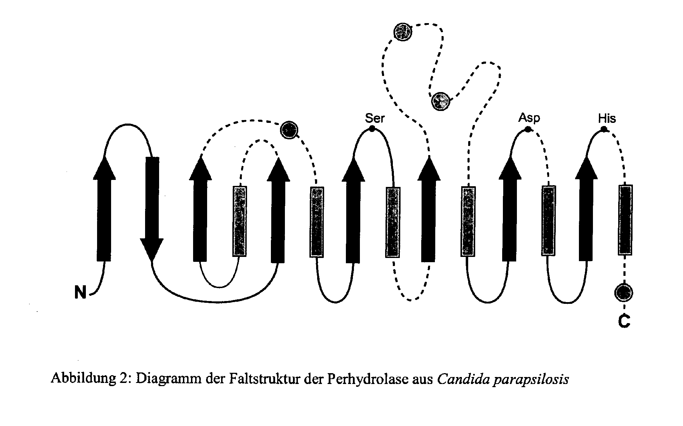

**Abbildung 2** zeigt das Diagramm der Faltstruktur der Perhydrolase aus Candida parapsilosis vom N-terminalen zum C-terminalem Ende. In der Abbildung bedeuten die grauen Abschnitte die alpha Helix des Enzyms, die schwarzen Pfeile entsprechen beta-Faltblatt und die benannten gekennzeichneten Aminosäuren entsprechen dem katalytischen Zentrum. Die gestrichelten Verbindungen entsprechen variablen Bereichen und die durchgezogenen Verbindungen beschreiben die konservierten Bereiche.

Durch Vergleich mit bekannten Enzymen, die beispielsweise in allgemein zugänglichen Datenbanken hinterlegt sind, lässt sich aus der Aminosäure- oder Nukleotid-Sequenz die enzymatische Aktivität eines betrachteten Enzyms folgern. Diese kann durch andere Bereiche des Proteins, die nicht an der eigentlichen Reaktion beteiligt sind, qualitativ oder quantitativ modifiziert werden. Dies könnte beispielsweise die Enzymstabilität, die Aktivität, die Reaktionsbedingungen oder die Substratspezifität betreffen.
Solch ein Vergleich geschieht dadurch, dass ähnliche Abfolgen in den Nukleotid- oder Aminosäuresequenzen der betrachteten Proteine einander zugeordnet werden. Dies nennt man Homologisierung. Eine tabellarische Zuordnung der betreffenden Positionen wird als alignment bezeichnet. Bei der Analyse von Nukleotidsequenzen sind wiederum beide komplementären Stränge und jeweils allen drei möglichen Leserastern zu berücksichtigen; ebenso die Degeneriertheit des genetischen Codes und die organismenspezifische Codon-Usage.

Eine Zusammenstellung aller in den verglichenen Sequenzen übereinstimmenden Positionen wird als Konsensus-Sequenz bezeichnet.
Solch ein Vergleich erlaubt auch eine Aussage über die Ähnlichkeit der verglichenen Sequenzen zueinander. Diese wird in Prozent Identität, das heißt dem Anteil der identischen Nukleotide oder Aminosäurereste an denselben Positionen wiedergegeben. Ein weiter gefasster Homologiebegriff bezieht die konservierten Aminosäure-Austausche in diesen Wert mit ein. Es ist dann von Prozent Homologie die Rede. Solche Aussagen können über ganze Proteine oder Gene oder nur über einzelne Bereiche getroffen werden.

Unter einem **Protein oder Polypeptid** ist im Sinne der vorliegenden Anmeldung ein aus den natürlichen Aminosäuren zusammengesetztes, weitgehend linear aufgebautes, zur Ausübung seiner Funktion zumeist dreidimensionale Struktur annehmendes Polymer zu verstehen. In der vorliegenden Anmeldung werden die 19 proteinogenen, natürlich vorkommenden L-Aminosäuren mit den international gebräuchlichen 1- und 3-Buchstaben-Codes bezeichnet.
Die Begriffe **Polypeptid und Polypeptide** werden im Sinne der Erfindung synonym verwendet und beinhalten jeweils die Bedeutung für die Einzahl wie für die Mehrzahl von Polypeptiden.

Unter einem **Enzym** ist im Sinne der vorliegenden Anmeldung ein Protein zu verstehen, das eine bestimmte biokatalytische Funktion ausübt.
Die Aminosäuresequenz des erfindungsgemäßen Enzyms wird im Sequenzprotokoll unter der Bezeichnung SEQ ID NO. 3 angegeben Die Aminosäuresequenz des erfindungsgemäßen Enzyms mit Signalpeptid wird unter der Bezeichnung SEQ ID NO 2 angegeben. Die Nukloetidsequenz dieses Enzyms mit Signalpeptid wird im Sequenzprotokoll unter der Bezeichnung SEQ ID NO. 1 angegeben. Sie steht somit für Weiterentwicklungen über an sich bekannte molekularbiologische Methoden zur Verfügung.

In einem weiteren Gegenstand der Erfindungen haben die beanspruchten Polypeptide mit Perhydrolase Aktivität eine Aminosäuresequenz von mindestens 200 Aminosäuren und zeigen nach Sequenzvergleich durch alignment mit der SEQ ID NO 3 in den überlappenden Bereichen der Aminosäuresequenzen wenigstens 80 % Homologie oder wenigstens 65 % Identität zur SEQ ID NO 3, mit Ausnahme der SEQ ID NO 3 selbst. Bevorzugt besitzt die Aminosäuresequenz mindestens 85 %, insbesondere mindestens 90 %, besonders bevorzugt mindestens 95 %, 96 %, 96,5 %, 97 %, 97,5 %, 98 %, 98,5 %, 99 % und ganz besonders bevorzugt 99,5 % Homologie **oder** mindestens 70 %, mindestens 75 %, mindestens 80 %, vorzugsweise mindestens 85 %, insbesondere mindestens 90 %, besonders bevorzugt mindestens 95 %, 96 %, 96,5 %, 97 %, 97,5 %, 98 %, 98,5 %, 99 % und ganz besonders bevorzugt 99,5 % Identität zur SEQ ID NO 3. Die Polypeptide mit Perhydrolase Aktivität mit einer Aminosäuresequenz von mindestens 200 Aminosäuren haben bevorzugt eine Aminosäuresequenz von mindestens 250 Aminosäuren, insbesondere mindestens 300 Aminosäuren. Hier sind Aminosäuresequenzen mit eingeschlossen, die im Vergleich zu den beschriebenen Polypeptiden nach SEQ ID NO 3 Sequenzbereiche aufweisen, die nicht übereinstimmen, da sie entweder in SEQ ID NO 3 nicht vorhanden sind also "gaps" enthalten, oder durch weitere Insertionen eine größere Anzahl an Aminosäuren aufweisen, jedoch einen überlappenden Bereich mit SEQ ID NO 3 zeigen. Die Homologie der gesamten zu vergleichenden Aminosäuresequenz kann dadurch kleiner als 80 % sein und die Identität kann geringer als 65% im Vergleich zur SEQ ID NO 3 sein, jedoch darf im überlappenden Bereich die 80 % Homologie oder die 65 % Identität nicht unterschritten werden.

Im Sinn der Anmeldung bedeutet ein Ausdruck der Form "mindestens X %" oder "wenigstens X %" oder "X % bis 99,5 %" einschließlich der Eckwerte X und 99,5 und aller ganzzahligen und nicht ganzzahligen Prozentwerte dazwischen.

Zahlreiche Proteine werden als so genannte **Präproteine** oder Präproproteine, also zusammen mit einem **Signalpeptid** und / oder Propeptid gebildet. Unter einem Signalpeptid ist der N-terminale Teil des Proteins zu verstehen, dessen Funktion darin besteht, die Ausschleusung des gebildeten Proteins aus der produzierenden Zelle in das Periplasma oder das umgebende Medium zu gewährleisten. Anschliessend wird das Signalpeptid unter natürlichen Bedingungen durch eine Signalpeptidase vom übrigen Proprotein abgespalten. Das Propeptid gewährleistet die korrekte Faltung des Proteins und wird vom reifen, katalytisch aktiven Protein abgespalten.
Für technische Anwendungen sind aufgrund ihrer enzymatischen Aktivität die maturen Peptide, das heißt die nach ihrer Herstellung prozessierten Enzyme gegenüber den Präproteinen bevorzugt.
Pro-Proteine sind inaktive Vorstufen von Proteinen. Deren Vorläufer mit Signalsequenz werden als Prä-Pro-Proteine bezeichnet.
Die SEQ ID NO 3 entspricht der Sequenz des maturen Peptides und ist mit Signalpeptid bestehend aus 16 Aminosäuren in der Anmeldung als SEQ ID NO 2 beschrieben. Im Stand der Technik ist sie als Polypeptid mit Lipase/Acyltransferase Aktivität aus der EP 1 275 711 bekannt.

In einem weiteren Gegenstand der Erfindung haben die Polypeptide mit Perhydrolase Aktivität mit einer Aminosäuresequenz, welche zu der in SEQ ID NO 3 angegebenen Aminosäuresequenz in den Positionen äquivalent zu **Positionen 170 bis 380** wenigstens 80% Homologie.

Ebenso im Sinne der Erfindung sind Polypeptide mit eingeschlossen, mit Perhydrolase Aktivität und einer Aminosäuresequenz, welche zu der in SEQ ID NO 3 angegebenen Aminosäuresequenz in den Positionen äquivalent zu **Positionen 1 bis 200 oder 330 bis 450** wenigstens 80 % Homologie aufweisen. Auch hier ist bevorzugt mindestens 83 %, mindestens 85 %, insbesondere mindestens 90 %, besonders bevorzugt mindestens 95 % und ganz besonders bevorzugt 99,5 % Homologie. Jeweils jedoch mit Ausnahme der SEQ ID NO 3. Besonders bevorzugt ist es, wenn jeweils wenigstens eine Aminosäure äquivalent zu Position **180, 332 und/oder 365** der SEQ ID NO 3 homolog ist. Insbesondere bevorzugt wenn wenigstens zwei Aminosäuren äquivalent zu Position **180, 332 und/oder 365** der SEQ ID NO 3 homolog sind und insbesondere bevorzugt, wenn drei Aminosäuren äquivalent zu Position **180, 332** und **365** der SEQ ID NO 3 homolog sind.

In einem weiteren Gegenstand der Erfindung haben die Polypeptide mit Perhydrolase Aktivität mit einer Aminosäuresequenz, welche zu der in SEQ ID NO 3 angegebenen Aminosäuresequenz in den Positionen äquivalent zu **Positionen 170 bis 380** wenigstens 65% Identität. Besonders bevorzugt ist eine 96 % Homologie oder eine 96 % Identität für die Teilbereiche in den Positionen äquivalent zu 170-190, 200 bis 270 und 310 bis 380.

Ebenso im Sinne der Erfindung sind Polypeptide mit eingeschlossen, mit Perhydrolase Aktivität und einer Aminosäuresequenz, welche zu der in SEQ ID NO 3 angegebenen Aminosäuresequenz in den Positionen äquivalent zu **Positionen 1 bis 200** oder **330 bis 450** wenigstens 65 % Identität aufweisen. Auch hier ist bevorzugt mindestens 70 %, mindestens 75 %, mindestens 80 %, vorzugsweise mindestens 85 %, insbesondere mindestens 90 %, besonders bevorzugt mindestens 95 % und ganz besonders bevorzugt 99,5 % Identität. Jeweils jedoch mit Ausnahme der SEQ ID NO 3.

Besonders bevorzugt ist es, wenn jeweils wenigstens eine Aminosäure äquivalent zu Position **180, 332 und/oder 365** der SEQ ID NO 3 identisch ist. Insbesondere bevorzugt wenn wenigstens zwei Aminosäuren äquivalent zu Position **180, 332 und/oder 365** der SEQ ID NO 3 identisch sind und insbesondere bevorzugt, wenn drei Aminosäuren äquivalent zu Position **180, 332 und 365** der SEQ ID NO 3 identisch sind.

In einer besonderen Ausführungsform der Erfindung sind die Polypeptide dadurch gekennzeichnet, dass sich wenigstens eine Modifikation äquivalent zu einer Position in der Aminosäuresequenz nach SEQ ID NO 3 befindet, die ausgewählt ist aus der Gruppe die gebildet wird aus:
Gly26, Ser27, Ile28, Leu29, Lys30, Thr31, Arg32, Val34, Asn36, Pro37, Leu38, Thr39, Asn40, Val41, Phe42, Thr43, Pro44, Lys46, Val47, Gln48, Asn49, Ala50, Trp51, GIn52, Leu53, Leu54, Val55, Arg56, Ser57, Asp59, Thr60, Lys79, Leu82, Val83, Ser84, Tyr85, Gln86, Thr87, Phe88, Glu89, Asp90, Ser91, Cys96, Pro98, Ser99, Tyr100, Tyr119, Thr132, Asp134, Tyr135, Pro138, Gln147, Ala151, Asn164 Leu176, Trp177, Gly178, Tyr179, Gly181, Gly182, Ser183, Leu184, Ala185, Ser186, Gly187, Trp188, Ala189, Glu199, Gly206, Ala207, Ala208, Leu209, Gly210, Gly211, Phe212, Val213, Thr214, Asn215, Ile216, Thr217, Glu221, Ala222, Asp224, Ser233, Asp245, Leu257, Leu258, Ser259, Ile260, Thr261, Tyr262, Arg263, Leu264, Gly265, Thr267, His268, Cys269, Leu270, Leu271, Asp272, Gly273, Phe278, Phe283, Ser284, Arg285, Ile286, Ile287, Arg288, Tyr289, Phe290, Pro291, Asp292, Gly293, Val297, Asn298, Gln299, Glu300, Pro301, Ile302, Lys303, Thr304, Asp308, Asn309, Asp317, Leu324, Phe325, Ile326, Tyr327, His328, Gly329, Thr330, Leu331, Ala333, Ile334, Val335, Pro336, Ile337, Val338, Asn339, Ser340, Arg341, Lys342, Thr343, Gln346, Trp347, Cys348, Glu356, Tyr357, Asn358, Glu359, Asp360, Leu361, Thr362, Asn363, Gly364, Ile366, Thr367, Glu368, Ser369, Ile370, Val371, Gly372, Ala373, Pro374, Leu377, Ile380, und Ile381.

Die hier gelisteten Aminosäuren befinden sich jeweils in einem räumlichen Abstand von weniger als 12 Å von der katalytischen Triade, die gebildet wird von Ser180, Asp332 und His365. Diese Aminosäuren wurden auf der Basis eines Homologie-Modelling mit der Chloroperoxidase aus *Pseudomonas fluorescens* ermittelt. Modifikation bedeutet im Sinne der Erfindung, dass wenigstens eine dieser Aminosäuren ausgetauscht sind.
Besonders bevorzugt ist wenigstens eine Modifikation äquivalent zu einer Position in der Aminosäuresequenz nach SEQ ID NO 3, die ausgewählt ist aus der Gruppe die gebildet wird aus:
Ser27, Ile28, Leu29, Lys30, Thr31, Arg32, Pro37, Leu38, Thr39, Asn40, Phe42, Thr43, Lys46, Val47, Gln48, Asn49, Ala50, Trp51, Gln52, Leu53, Val55, Asp59, Lys79, Ser84, Tyr85, Gln86, Thr87, Phe88, Glu89, Asp90, Pro98, Ser99, Tyr100, Tyr135, Gln147, Trp177, Gly178, Tyr179, Gly181, Gly182, Ser183, Leu184, Ala185, Ser186, Gly187, Trp188, Ala207, Ala208, Leu209, Gly210, Gly211, Phe212, Val213, Thr214, Asn215, Ile216, Thr217, Leu258, Ser259, Ile260, Thr261, Tyr262, Arg263, Leu264, Thr267, His268, Cys269, Leu271, Asp272, Phe278, Phe283, Ser284, Ile286, Ile287, Arg288, Tyr289, Phe290, Pro291, Val297, Gln299, Glu300, Pro301, Ile302, Thr304, Tyr327, His328, Gly329, Thr330, Leu331, Ala333, Ile334, Val335, Pro336, Ile337, Val338, Asn339, Ser340, Arg341, Thr343, Glu356, Asn358, Glu359, Asp360, Leu361, Thr362, Asn363, Gly364, Ile366, Thr367, Glu368, Ser369, Ile370, Val371, Leu377.
Die in dieser bevorzugten Gruppe gelisteten Aminosäuren befinden sich jeweils in einem räumlichen Abstand von weniger als 10 Å von der katalytischen Triade, die gebildet wird von Ser180, Asp332 und His365.

Insbesondere bevorzugt ist wenigstens eine Modifikation äquivalent zu einer Position in der Aminosäuresequenz nach SEQ ID NO 3, die ausgewählt ist aus der Gruppe die gebildet wird aus:
11e28, Leu29, Lys30, Asn49, Ala50, Trp51, Gln52, Tyr85, Gln86, Thr87, Phe88, Tyr100, Tyr135, Gly178, Tyr179, Gly181, Gly182, Ser183, Leu184, Ala185, Leu209, Gly210, Gly211, Phe212, Val213, Ile260, Thr261, Tyr262, Ile287, Arg288, Tyr289, Glu300, His328, Gly329, Thr330, Leu331, Ala333, Ile334, Val335, Pro336, Ile337, Thr362, Asn363, Gly364, Ile366, Thr367, Glu368.
Die in dieser bevorzugten Gruppe gelisteten Aminosäuren befinden sich jeweils in einem räumlichen Abstand von weniger als 6 Å von der katalytischen Triade, die gebildet wird von Ser180, Asp332 und His365.

Die Positionen, an denen das Polypeptid glycosyliert vorliegt und der Grad der Glycosylierung sind abhängig vom Organismus der dieses Polypeptid produziert. Bevorzugt ist eine Glycosylierung an einer Position äquivalent zur Position Asn164 in der Aminosäuresequenz SEQ ID NO 3. Es ist bevorzugt, dass bei den genannten Modifikationen die Position äquivalent zu Position 164 in SEQ ID NO 3 nicht modifiziert wird.

Im Sinne der Erfindung wird der Begriff "katalytische Triade" synonym verwendet zu dem Begriff **"aktives Zentrum"** oder "katalytisches Zentrum" des Polypeptides und betrifft den Bereich des Polypeptides, in dem die Bindung der Substrate und deren katalytischer Umsatz zu den Produkten stattfindet. Das katalytische Zentrum muss einen hohen Grad an Substratspezifität besitzen und in der Lage sein, einen aktiven Übergangskomplex mit dem Substrat zu bilden.

In einer bevorzugten Ausführungsform der Erfindung handelt es sich bei den beanspruchten Polypeptiden mit Perhydrolase Aktivität um **strukturelle Homologe**, bei denen das aktive Zentrum identisch ist zu wenigstens einer Aminosäure die äquivalent ist zu Positionen in der Aminosäuresequenz der SEQ ID NO 3 die ausgewählt sind aus der Gruppe die gebildet wird aus S 180, D332 und H365 der SEQ ID NO 3.

Im Sinne der Erfindung versteht man unter strukturellen Homologen die Homologe bezüglich der Topologie also der räumlichen Struktur bzw. der Tertiärstruktur des Proteins. Diese strukturellen Homologen können ermittelt werden durch alignment von Proteinen mittels spezifischen Algorithmen, die eine ähnliche räumliche Struktur vorhersagen können.

Der Grenzwert einer Sequenz Homologie, die zu einer strukturellen Homologie führt, hängt ab von der Länge des vergleichenden alignments: Bei einer alignment Länge von 50 Aminosäuren bei der also zwei Polypeptide mit jeweils 50 Aminosäuren verglichen werden, benötigt man eine 32,3 % Identität der Aminosäuresequenzen damit eine strukturelle Homologie vorliegen kann. Bei einem Sequenzvergleich von 60 Aminosäuren genügt eine Identität von 29,1 % und bei einem Sequenzvergleich von größer oder gleich 80 Aminosäuren liegt der Grenzwert der notwendigen Identität bei 24,8 %. Diese Grenzwerte t(L) sind einer Analyse von tausenden von alignments von der Strukturdatenbank (PDT) entnommen und können dargestellt werden durch die Formel: t(L) = 290,15L ^{-0,562} wobei L bestimmt wurde für die Länge der Aminosäuresequenz zwischen 10 und 80. Bei mehr als 80 Aminosäuren liegt der Grenzwert generell bei 25 % Identität der Aminosäuresequenzen.

### Motife:

In einem weiteren Gegenstand der Erfindung enthalten die Polypeptide mit Perhydrolase Aktivität wenigstens ein Motif, welches wenigstens 50 % Homologie zeigt zu einem Motif ausgewählt aus der Gruppe, die gebildet wird aus
SEQ ID NO 4: GYSGGxxAxxWAxxxxxxYAPE,
SEQ ID NO 5: GYSGGxxAxxWAxxxxxxYAPD,
SEQ ID NO 6: GFSGGxxAxxWAxxxxxxYAPE,
SEQ ID NO 7: GFSGGxxAxxWAxxxxxxYAPD,
SEQ ID NO 8: GYSGGxxAxxWAxxxxxxYA und
SEQ ID NO 9: GFSGGxxAxxWAxxxxxxYA.
x steht dabei jeweils für eine essentielle oder nichtessentielle Aminosäure. In dem 3-Buchstaben-Code beispielsweise wie im Sequenzprotokoll wird diese Aminosäure als Xaa dargestellt und bedeutet auch dort jeweils eine essentielle oder nichtessentielle Aminosäure.

Bevorzugt enthalten diese Polypeptide Motife mit mindestens 55%, 60%, 65 %, 70 %, 75 %, oder mindestens 80 %, vorzugsweise mindestens 85 %, insbesondere mindestens 90 %, besonders bevorzugt mindestens 95 % und ganz besonders bevorzugt 99,5 % Homologie zu den genannten Motifen.

In einem weiteren Gegenstand der Erfindung enthalten die Polypeptide mit Perhydrolase Aktivität wenigstens ein Motif, welches wenigstens 70 % Identität zeigt zu einem Motif ausgewählt aus der Gruppe, die gebildet wird aus
SEQ ID NO 4: GYSGGxxAxxWAxxxxxxYAPE,
SEQ ID NO 5: GYSGGxxAxxWAxxxxxxYAPD,
SEQ ID NO 6: GFSGGxxAxxWAxxxxxxYAPE,
SEQ ID NO 7: GFSGGxxAxxWAxxxxxxYAPD,
SEQ ID NO 8: GYSGGxxAxxWAxxxxxxYA und
SEQ ID NO 9: GFSGGxxAxxWAxxxxxxYA.
x steht dabei jeweils für eine essentielle oder nichtessentielle Aminosäure. In dem 3-Buchstaben-Code beispielsweise wie im Sequenzprotokoll wird diese Aminosäure als Xaa dargestellt und bedeutet auch dort jeweils eine essentielle oder nichtessentielle Aminosäure.

Bevorzugt enthalten diese Polypeptide Motife mit mindestens 75 % oder mindestens 80 %, vorzugsweise mindestens 85 %, insbesondere mindestens 90 %, besonders bevorzugt mindestens 95 % und ganz besonders bevorzugt 99,5 % Identität zu den genannten Motifen.

Ebenso bevorzugt sind Polypeptide mit Perhydrolase Aktivität, welche Motife mit den genannten Homologien oder Identitäten zu den ausgewählten Motifen zeigen und zu den Polypeptiden mit Perhydrolase Aktivität zählen, die mit einer Aminosäuresequenz, welche zu der in SEQ ID NO 3 angegebenen Aminosäuresequenz wenigstens 80 % Homologie besitzen, mit Ausnahme der SEQ ID NO 3 oder die mit einer Aminosäuresequenz, welche zu der in SEQ ID NO 3 angegebenen Aminosäuresequenz wenigstens 65 % Identität besitzen, mit Ausnahme der SEQ ID NO 3. Die bevorzugten Homologien oder Identitäten gelten analog den oben genannten in Bezug auf Motife.

Ebenso bevorzugt sind Polypeptide mit Perhydrolase Aktivität, welche Motife mit den genannten Homologien oder Identitäten zu den ausgewählten Motifen zeigen und zu den Polypeptiden mit Perhydrolase Aktivität zählen, die eine Aminosäuresequenz von mindestens 200 Aminosäuren besitzen und nach Sequenzvergleich durch alignment mit der SEQ ID NO 3 in den **überlappenden Bereichen** der Aminosäuresequenzen wenigstens 80 % Homologie oder wenigstens 65 % Identität zur SEQ ID NO 3, mit Ausnahme der SEQ ID NO 3 zeigen.

Ebenso bevorzugt sind Polypeptide mit Perhydrolase Aktivität, welche Motife mit den genannten Homologien oder Identitäten zu den ausgewählten Motifen zeigen und zu den Polypeptiden mit Perhydrolase Aktivität zählen, die eine Aminosäuresequenz aufweisen, welche zu der in SEQ ID NO 3 angegebenen Aminosäuresequenz in den Positionen äquivalent zu **Positionen 170 bis 380** wenigstens 80 % Homologie oder wenigstens 65 % Identität zeigen.

In einer besonderen Ausführungsform sind die erfindungsgemäßen Polypeptide mit Perhydrolase Aktivität dadurch gekennzeichnet, dass es sich um Polypeptide aus der Familie der α/β-Hydrolasen handelt.

In einer besonderen Ausführungsform handelt es sich bei den erfindungsgemäßen Polypeptiden mit Perhydrolase Aktivität gemäß der oben beschriebenen Polypeptide um Derivate, erhältlich durch Derivatisierung, Verknüpfung, Fragmentierung, Deletionsmutation, Insertionsmutation oder Punktmutation.

Unter **Fragmenten** werden alle Proteine oder Peptide verstanden, die kleiner sind als natürliche Proteine, die kleiner sind als jene Proteine, die denen von SEQ ID NO. 3 entsprechen, zu ihnen in den entsprechenden Teilsequenzen aber hinreichend homolog sind, oder solche, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder hydrolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Bei den Fragmenten kann es sich beispielsweise um einzelne Domänen handeln oder um Bruchstücke, die nicht mit den Domänen übereinstimmen. Solche Fragmente können kostengünstiger herzustellen sein, bestimmte eventuell nachteilige Charakteristika des Ausgangsmoleküls nicht mehr besitzen, wie möglicherweise einen aktivitätssenkenden Regulationsmechanismus, oder ein günstigeres Aktivitätsprofil entfalten. Derartige Proteinfragmente können auch nicht-biosynthetisch, sondern beispielsweise chemisch hergestellt werden. Die chemische Synthese kann beispielsweise dann vorteilhaft sein, wenn im Anschluß an die Synthese chemische Modifikationen vorgenommen werden sollen.

Den Fragmenten sind wegen ihrer prinzipiellen Gleichartigkeit auch durch Deletionsmutation erhältliche Polypeptide zuzuordnen. Solche können biochemisch weitgehend mit den Ausgangsmolekülen übereinstimmen oder einzelne Funktionen gerade nicht mehr aufweisen. Dies erscheint beispielsweise bei der Deletion inhibierender Bereiche besonders sinnvoll. Im Ergebnis können mit den Deletionen sowohl eine Spezialisierung als auch eine Erweiterung des Anwendungsbereichs des Proteins einhergehen. Sofern dadurch eine im weitesten Sinne Perhydrolase Aktivität aufrechterhalten, modifiziert, spezifiziert oder auch erst erreicht wird, handelt es sich bei den durch Deletionsvarianten wie bei den Fragmenten um erfindungsgemäße Proteine; einzige zusätzliche Voraussetzung dafür ist, dass sie über die noch vorhandene homologe Teilsequenz hinweg innerhalb des angegebenen Homologiebereichs zu der Sequenz SEQ ID NO. 3 liegen.

Unter durch **Insertionsmutation** erhaltene Polypeptide sind solche zu verstehen, die über an sich bekannte Methoden durch Einfügen eines Nucleinsäure-, beziehungsweise Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten Proteinen ist der Anteil an Fremdprotein geringer als in chimären Proteinen.

Inversionsmutagenese, also eine partielle Sequenzumkehrung, kann als Sonderform sowohl der Deletion, als auch der Insertion angesehen werden. Dasselbe gilt für eine von der ursprünglichen Aminosäureabfolge abweichende Neugruppierung verschiedener Molekülteile. Sie kann sowohl als Deletionsvariante, als Insertionsvariante, als auch als Shuffling-Variante des ursprünglichen Proteins angesehen werden.

Unter **Derivaten** werden im Sinne der vorliegenden Anmeldung solche Polypeptide verstanden, deren reine Aminosäurekette chemisch modifiziert worden ist. Solche Derivatisierungen können beispielsweise biologisch im Zusammenhang mit der Proteinbiosynthese durch den Wirtsorganismus erfolgen. Hierfür können molekularbiologische Methoden eingesetzt werden. Sie können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise um andere Proteine handeln, die beispielsweise über bifunktionelle chemische Verbindungen an erfindungsgemäße Polypeptide gebunden werden. Ebenso ist unter Derivatisierung die kovalente Bindung an einen makromolekularen Träger zu verstehen. Derartige Modifikationen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Eine weitere Ausführungsform sind deshalb solche Derivate, die durch kovalente Bindung an einen makromolekularen Träger, wie beispielsweise Polyethylenglykol oder ein Polysaccharid erhalten worden sind.
Im Sinne der vorliegenden Erfindung werden alle Polypeptide, Enzyme, Proteine, Fragmente und Derivate, sofern sie nicht explizit als solche angesprochen zu werden brauchen, unter dem Oberbegriff Polypeptide zusammengefasst.

Die enzymatische Aktivität kann durch andere Bereiche des Polypeptides, die nicht an der eigentlichen Reaktion beteiligt sind, qualitativ oder quantitativ modifiziert werden. Dies betrifft beispielsweise die Enzymstabilität, die Aktivität, die Reaktionsbedingungen oder die Substratspezifität. Denn einerseits ist nicht genau bekannt, welche Aminosäurereste der erfindungsgemäßen Polypeptide tatsächlich die Hydrolyse, Transesterifizierung und Veresterung katalysieren, und andererseits können nicht von vornherein bestimmte Einzelfunktionen definitiv von der Beteiligung an der Katalyse ausgenommen werden. Zu den Hilfsfunktionen oder Teilaktivitäten gehören beispielsweise die Bindung eines Substrats, eines Zwischen- oder Endprodukts, die Aktivierung oder die Inhibierung oder Vermittlung eines regulierenden Einflusses auf die hydrolytische Aktivität. Dabei kann es sich beispielsweise auch um die Ausbildung eines Strukturelements handeln, das fern vom aktiven Zentrum liegt, oder um ein Signalpeptid, dessen Funktion die Ausschleusung des gebildeten Proteins aus der Zelle und/oder dessen korrekte Faltung betrifft und ohne das in vivo in der Regel kein funktionsfähiges Enzym gebildet wird. Allerdings muss sich insgesamt eine Hydrolyse, Transesterifizierung und Veresterung katalysiert werden.

Proteine können auch über die Reaktion mit einem Antiserum oder einem bestimmten Antikörper zu Gruppen immunologisch verwandter Proteine zusammengefasst werden. Die Angehörigen einer Gruppe zeichnen sich dadurch aus, dass sie dieselbe, von einem Antikörper erkannte antigene Determinante aufweisen
Eine weitere Ausführungsform der Erfindung sind Polypeptide oder Derivate, die wenigstens 4 antigene Determinanten mit einem der oben genannten erfindungsgemäßen Polypeptide oder Derivate gemeinsam haben, die also übereinstimmen. Bevorzugt sind wenigstens 5 bis 20 übereinstimmende antigene Derterminaten, besonders bevorzugt sind wenigstens 10 bis 20 übereinstimmende antigene Determinaten und insbesondere wenigstens 15 bis 18 übereinstimmende antigene Determinaten.
Denn entscheidend für die Ausübung enzymatischer Aktivitäten ist nicht allein die pure Aminosäureabfolge eines Proteins, sondern auch dessen Sekundärstrukturelemente und dessen dreidimensionale Faltung (Tertiärstruktur). So können in ihrer Primärstruktur deutlich voneinander abweichende Domänen räumlich weitgehend übereinstimmende Strukturen ausbilden und somit gleiches enzymatisches Verhalten ermöglichen. Solche Gemeinsamkeiten in der Sekundärstruktur werden üblicherweise als übereinstimmende **antigene Determinanten** von Antiseren oder reinen oder monoklonalen Antikörpern erkannt. Über immunchemische Kreuzreaktionen können somit einander strukturell ähnliche Proteine oder Derivate detektiert und zugeordnet werden.
Deshalb werden in den Schutzbereich der vorliegenden Erfindung gerade auch solche Polypeptide oder Derivate einbezogen, die Perhydrolase Aktivität aufweisen und möglicherweise nicht über ihre Homologiewerte in der Primärstruktur, wohl aber über ihre immunchemische Verwandtschaft den oben definierten erfindungsgemäßen Proteinen oder Derivaten zugeordnet werden können.

In einer besonderen Ausführungsform handelt es sich bei den erfindungsgemäßen Polypeptiden mit Perhydrolase Aktivität gemäß der oben beschriebenen Polypeptide um solche, die ein **Perhydrolyse zu Hydrolyse Verhältnis** bei Temperaturen zwischen 20 °C und 30 °C von größer oder gleich 0,1 zeigen. Bevorzugt ist ein Perhydrolyse zu Hydrolyse Verhältnis von größer oder gleich 0,4; 1; 1,5; 2; 2,5; 3, 3,5; oder 4. Besonders bevorzugt ist das Perhydrolyse zu Hydrolyse Verhältnis so groß wie möglich und insbesondere bis zu 9:1.

Dieses Verhältnis wird beispielsweise dadurch bestimmt, dass in Konzentrationsreihen durch GC-Analyse der Gehalt an freigesetzten Säuren und der Gehalt an Persäuren durch Absorptionsmessung nach Reaktion mit ABTS analysiert wird.
Hierzu wird 90 *µg* Polypeptid bei pH = 6,5 mit Dekansäuremethylester mit unterschiedlichen Zugaben an Wasserstoffperoxid inkubiert und nach 15 min Reaktionszeit die Konzentration an Persäure bestimmt. In diesen Versuchsreihen konnte festgestellt werden, dass die erfindungsgemäßen Polypeptide mit einer Aminosäuresequenz von mindestens 99,5 % Identität mit SEQ ID NO 3 sehr hohe Stabilität zeigen bei Wasserstoffperoxidkonzentrationen von bis zu 11 % bei Reaktionstemperaturen von 30 °C oder 45 °C.

Besonders bevorzugt sind Polypeptide mit Perhydrolase Aktivität gemäß der oben beschriebenen Polypeptide, die gegenüber dem Wildtyp-Enzym entsprechend einem Enzym mit einer Aminosäuresequenz mit 100 % Identität zu SEQ ID NO 3 ein um mindestens 20 % erhöhtes Perhydrolyse zu Hydrolyse Verhältnis aufweisen.

Das pH-Optimum für die katalytische Reaktion der Perhydrolyse welche bei 28 °C bestimmt wurde, liegt zwischen 3 und 10, bevorzugt zwischen 4 und 9, insbesondere zwischen 6 und 7,5 und besonders bevorzugt bei 6,5.
Bei Enzymmutanten für die Waschmittelapplikation werden besonders solche Mutanten bevorzugt, deren katalytisches pH-Optimum in Richtung höherer pH-Werte verschoben ist. Bevorzugt liegt das pH-Optimum dann zwischen 6,5 und 11, insbesondere zwischen 7,5 und 10.

Ein optimaler Temperaturbereich bei der Perhydrolyse des Wildtyp Enzyms, bestimmt beim pH-Optimum von 6,5, liegt zwischen 10 und 60 °C, bevorzugt zwischen 20 und 45 °C. Ein optimaler Temperaturbereich für die Katalyse der erfindungsgemäßen Perhyrdrolyse, bestimmt beim pH-Optimum von 6,5, liegt zwischen 20 bis 60 °C, bevorzugt zwischen 30 und 45 °C.

In einer weiteren Ausführungsform der Erfindung werden Polypeptide mit Perhydrolase Aktivität gemäß den oben beschriebenen Polypeptiden dadurch gekennzeichnet, dass sie eine optimale Stabilität und/oder optimale thermische Stabilität und/oder einen optimalen Verlust der enzymatischen Katalyseleistung und/oder eine optimale chemische Stabilität und/oder eine optimale pH-Stabiliät im Vergleich zum Wild-typ Polypeptid mit Perhydrolase Aktivität gemäß SEQ ID NO 2 oder SEQ ID NO 3 aufweisen.

Die optimalen Stabilitäten hängen vom verwendeten Anwendungssystem ab. Im folgenden wird das selbstkontrollierende System beschrieben. Gewünscht ist nämlich kein absolut stabiles Enzym, das bei hohen Temperaturen zu einer zu raschen und zu hohen Persäurekonzentration und damit zu Schädigungen führen würde. Optimal heißt, dass genügend Persäure im gewünschten Temperaturbereich bei Enzymkonzentrationen von kleiner 5% gebildet werden kann.
Die Methoden zur Bestimmung dieser Eigenschaften sind dem Fachmann hinlänglich bekannt.
Thermostabilität bedeutet in dem Zusammenhang, dass die Polypeptide in einem weiten Temperaturbereich Aktivität aufweisen. Sie wird bestimmt über Halbwertszeiten der relevanten Aktivitäten nach einer Inkubation der Enzyme. Enzyme zeigen eine Aktivität in einem weiten pH-Bereich. Die meisten Enzyme zeigen einen bestimmten pH-Wert an dem die Aktivität ein Optimum aufweist. Viele Enzyme zeigen im extrem basischen oder extrem sauren Bereich keine Aktivität mehr. Die pH-Stabilitäten sind Stabilitätswerte, die auf einen bestimmten pH-Wert bezogen sind. Dabei müssen pH-Aktivitäts-Optima und pH-Stabilitäts-Optima nicht übereinstimmen.
Enzymatische und chemische Stabilität ist bezogen auf andere Enzyme oder Chemikalien, die die Aktivität der erfindungsgemäßen Polypeptide mit Perhydrolase Aktivität herabsetzen könnten. Eine erhöhte Stabilität bezieht sich auf eine verlängerte oder verbesserte Aktivität der erfindungsgemäßen Polypeptide in Gegenwart von schädigenden Enzymen oder Chemikalien. Hier sind alle Enzyme oder Chemikalien miteinbezogen, die die Aktivität der erfindungsgemäßen Polypeptide herabsetzen könnten.

Erfindungsgemäße Polypeptide, die aus natürlichen Quellen stammen, sind bevorzugte Ausführungsformen der vorliegenden Erfindung, insbesondere wenn sie aus Mikroorganismen wie einzellige Pilze oder Bakterien stammen. Denn diese lassen sich zumeist einfacher handhaben als vielzellige Organismen oder die von Vielzellern abgeleiteten Zellkulturen. Diese stellen für spezielle Ausführungsformen sinnvolle Optionen dar.

Besonders bevorzugt sind erfindungsgemäße Polypeptide oder Derivate aus eukaryontischen Pilzen, insbesondere diese, die sekretierte Proteine unmittelbar ins umgebende Medium abgeben können.
Besonders bevorzugt sind erfindungsgemäße Polypeptide oder Derivate die erhältlich sind aus Mikroorganismen die Species angehören die ausgewählt sind aus der Gruppe, die gebildet wird von *Candida, Kluyveromyces, Geotrichum, Fusarium, Aeromonas, Debaryomyces, Arxula, Zygoascus, Emercilla, Aspergillus, Malassezia, Gibberella, Kurzmanomyces, Ustillago, Nocardia, Cordyceps, Mycobacterium, Rhodococcus, Saccharopolyspora, Burkholderia, Corynebacterium, Saccharopolyspora, Pseudomonas, Streptomyces* und *Pichia.*
Ganz besonders bevorzugt sind erfindungsgemäße Polypeptide oder Derivate die erhältlich sind aus Mikroorganismen, die ausgewählt sind aus der Gruppe, die gebildet wird von *Candida parapsilosis, Candida antarctica (Trychosporon oryzae, Pseudozyma antarctica) Candida glabrata, Candida albicans, Candida maltosa, Candida tropicalis, Candida viswanathii, Issatchenkia orientalis (Candida krusei), Kluyveromyces marxianus (C. kefyr, C. pseudotropicalis), Pichia guilliermondii (Candida guilliermondii), Geotrichum candidum, Fusarium solani Aeromonas aerophila, Debaryomyces hansenii, Arxula adeninivorans, Zygoascus hellenicus, Aspergillus fumigatus, Emercilla nidulans, Malassezia pachydermatis, Gibberella zeae, Aspergillus niger, Fusarium sporotrichioides, Kurzmanomyces sp., Ustillago maydis, Nocardia farcinica, Cordyceps bassania, Mycobacterium tuberculosis, Mycobacterium bovis, Mycobacterium avium, Rhodococcus sp., Saccharopolyspora spinosa, Burkholderia cenocepacia, Corynebacterium diphtheriae, Pseudomonas fluorescens Saccharopolyspora pogona,* und *Streptomyces coelicolor.*

Unter den erfindungsgemäßen Polypeptiden oder Derivate aus *Candida-Species* wiederum, sind die aus *Candida parapsilosis* oder *Candida albicans* bevorzugt, darunter insbesondere aus *Candida parapsilosis* CBS 604, denn aus diesem wurde die Ausführungsform des erfindungsgemäßen Enzyms, deren zugehörige Sequenzen im Sequenzprotokoll unter SEQ ID NO 2 und SEQ ID NO 3 angegeben sind, ursprünglich erhalten.
Aus produktionstechnischen Gründen sind jeweils solche Stämme bevorzugt, die das gebildete Polypeptid in das sie umgebende Medium abgeben.

Ein weiterer Gegenstand der Erfindung sind Nucleinsäuren, die für ein Polypeptid mit Perhydrolase Aktivität codieren, deren Nucleotidsequenz zu der in SEQ ID NO. 1 angegebenen Nucleotidsequenz wenigstens 80 % Homologie aufweisen mit Ausnahme der SEQ ID NO 1, oder 65 % Identität zeigen mit Ausnahme der SEQ ID NO 1. Insbesondere solche Nucleotidsequenzen, die wenigstens 80 % Homologie oder 65 % Identität zeigen über den Teilbereich, der für die Aminosäuren äquivalent zu Positionen 170 bis 380 aus der SEQ ID NO 3 codiert.

Weiterhin sind Nucleinsäuren beansprucht, codierend für eine Aminosäuresequenz, welche zu der in SEQ ID NO 2 oder SEQ ID NO 3 angegebenen Aminosäuresequenz wenigstens 85%, bevorzugt 90 %, besonders bevorzugt 95 % und insbesondere 99,5 % Homologie besitzt.
Ebenso sind Nucleinsäuren beansprucht, codierend für eine Aminosäuresequenz, welche zu der in SEQ ID NO 2 oder SEQ ID NO 3 angegebenen Aminosäuresequenz wenigstens 70 %, 75 % oder 85 %, bevorzugt 90 %, besonders bevorzugt 95 % und insbesondere 99,5 % Identität besitzt.
Bevorzugt sind Nucleinsäuren, kodierend für eine Aminosäuresequenz, welche zu der in SEQ ID NO. 2 angegebenen Aminosäuresequenz äquivalent zu den Positionen 190 bis 390 oder zu der in SEQ ID NO 3 angegebenen Aminosäuresequenz äquivalent zu den Positionen 170 bis 380 mindestens zu 96 % identisch sind. Insbesondere bevorzugt sind Nucleinsäuren, die für eines der oben beschriebenen Polypeptide oder Derivate codieren. Der Ähnlichkeitsbereich umfasst auch alle Polypeptide, deren Nucleotidsequenz zu der in SEQ ID NO. 1 angegebenen Nucleotidsequenz mindestens zu 85 %, zu 87,5 %, zu 90 %, zu 92,5 %, zu 95 %, zu 96 %, zu 97 %, zu 98 %, zu 99 % oder zu 99,5 % identisch ist.

Unter **Nucleinsäuren** sind im Sinne der vorliegenden Anmeldung die natürlicherweise aus Nucleotiden aufgebauten als Informationsträger dienenden Moleküle zu verstehen, die für die lineare Aminosäureabfolge in Proteinen oder Enzymen codieren. Sie können als Einzelstrang, als ein zu diesem Einzelstrang komplementärer Einzelstrang oder als Doppelstrang vorliegen. Als der natürlicherweise dauerhaftere Informationsträger ist die Nucleinsäure DNA für molekularbiologische Arbeiten bevorzugt. Demgegenüber wird für die Realisierung der Erfindung in natürlicher Umgebung, wie beispielsweise in einer exprimierenden Zelle, eine RNA gebildet, weshalb erfindungswesentliche RNA-Moleküle ebenfalls Ausführungsformen der vorliegenden Erfindung darstellen.
Bei DNA sind die Sequenzen beider komplementärer Stränge in jeweils allen drei möglichen Leserastern zu berücksichtigen. Ferner ist zu berücksichtigen, dass verschiedene Codon-Triplets für dieselben Aminosäuren codieren können, so dass eine bestimmte AminosäureAbfolge von mehreren unterschiedlichen und möglicherweise nur geringe Identität aufweisenden Nucleotidsequenzen abgeleitet werden kann (Degeneriertheit des genetischen Codes). Außerdem weisen verschiedene Organismen Unterschiede im Gebrauch dieser Codons auf. Aus diesen Gründen müssen sowohl Aminosäuresequenzen als auch Nukleotidsequenzen in die Betrachtung des Schutzbereichs einbezogen werden und angegebene Nukleotidsequenzen sind jeweils nur als eine beispielhafte Codierung für eine bestimmte Aminosäurefolge anzusehen.

Die einem Protein entsprechende Informationseinheit wird auch im Sinne der vorliegenden Anmeldung als Gen bezeichnet.
Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen.

Änderungen der Nukleotidsequenz, wie sie beispielsweise durch an sich bekannte molekularbiologische Methoden herbeigeführt werden können, werden als Mutationen bezeichnet. Je nach Art der Änderung kennt man beispielsweise Deletions-, Insertions- oder Substitutionsmutationen oder solche, bei denen verschiedene Gene oder Teile von Genen miteinander fusioniert (shuffling) werden; dies sind Genmutationen. Die zugehörigen Organismen werden als Mutanten bezeichnet. Die von mutierten Nukleinsäuren abgeleiteten Proteine werden als Varianten bezeichnet. So führen beispielsweise Deletions-, Insertions-Substitutionsmutationen oder Fusionen zu deletions-, insertions- substitutionsmutierten oder Fusionsgenen und auf Proteinebene zu entsprechenden Deletions-, Insertions- oder Substitutionsvarianten, beziehungsweise Fusionsproteinen.
Der hier verwendete Ausdruck "Identität" in Bezug auf die Aminosäuresequenz bezeichnet eine Identität zur gegebenen Aminosäuresequenz die dazu führt, dass das Polypeptid mit einer angegebenen Identität die gleiche biologische Aktivität besitzt wie das erste Polypeptid. Die Identität der **Nucleotidsequenz** bezieht sich auf zu einer ersten Nucleotidsequenz homologes Gen. Homolog bedeutet in Bezug auf die Nucleotidsequenz, dass das Gen allelisch sein kann. Homolog in Bezug auf Nucleinsäuresequenz bedeutet ferner, dass das Gen aus einer anderen Species stammen kann, das von diesem Gen codierte Polypeptid aber die gleiche biologische Aktivität besitzt wie das von der ersten Nucleotidsequenz codierte Polypeptid.

Unter Vektoren werden im Sinne der vorliegenden Erfindung aus Nukleinsäuren bestehende Elemente verstanden, die als kennzeichnenden Nukleinsäurebereich ein interessierendes Gen enthalten. Sie vermögen dieses in einer Spezies oder einer Zellinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Man unterscheidet in der Gentechnik einerseits zwischen solchen Vektoren, die der Lagerung und somit gewissermaßen auch der gentechnischen Arbeit dienen, den so genannten Klonierungsvektoren, und andererseits denen, die die Funktion erfüllen, das interessierende Gen in der Wirtszelle zu realisieren, das heißt, die Expression des betreffenden Proteins zu ermöglichen. Diese Vektoren werden als Expressionsvektoren bezeichnet.

Im Rahmen der vorliegenden Erfindung wird die Nukleinsäure geeigneterweise in einen Vektor kloniert. Die molekularbiologische Dimension der Erfindung besteht somit in Vektoren mit den Genen für die entsprechenden Proteine.
Dazu können beispielsweise solche gehören, die sich von bakteriellen Plasmiden, von Viren oder von Bacteriophagen ableiten, oder überwiegend synthetische Vektoren oder Plasmide mit Elementen verschiedenster Herkunft. Mit den weiteren jeweils vorhandenen genetischen Elementen vermögen Vektoren, sich in den betreffenden Wirtszellen über mehrere Generationen hinweg als stabile Einheiten zu etablieren. Es ist dabei im Sinne der Erfindung unerheblich, ob sie sich extrachromosomal als eigene Einheiten etablieren oder in ein Chromosom integrieren. Welches der zahlreichen aus dem Stand der Technik bekannten Systeme gewählt wird, hängt vom Einzelfall ab. Ausschlaggebend können beispielsweise die erreichbare Kopienzahl, die zur Verfügung stehenden Selektionssysteme, darunter vor allem Antibiotikaresistenzen, oder die Kultivierbarkeit der zur Aufnahme der Vektoren befähigten Wirtszellen sein.
Die Vektoren bilden geeignete Ausgangspunkte für molekularbiologische und biochemische Untersuchungen des betreffenden Gens oder zugehörigen Proteins und für erfindungsgemäße Weiterentwicklungen und letztlich für die Amplifikation und Produktion erfindungsgemäßer Polypeptide. Sie stellen insofern Ausführungsformen der vorliegenden Erfindung dar, als die Sequenzen der enthaltenen erfindungsgemäßen Nukleinsäurebereiche jeweils innerhalb der oben näher bezeichneten Homologiebereiche liegen.

Bevorzugte Ausführungsformen der vorliegenden Erfindung sind Klonierungsvektoren. Diese eignen sich neben der Lagerung, der biologischen Amplifikation oder der Selektion des interessierenden Gens für die Charakterisierung des betreffenden Gens, etwa über das Erstellen einer Restriktionskarte oder die Sequenzierung. Klonierungsvektoren sind auch deshalb bevorzugte Ausführungsformen der vorliegenden Erfindung, weil sie eine transportierbare und lagerfähige Form der beanspruchten DNA darstellen. Sie sind auch bevorzugte Ausgangspunkte für molekularbiologische Techniken, die nicht an Zellen gebunden sind, wie beispielsweise die Polymerasekettenreaktion.
Expressionsvektoren sind chemisch den Klonierungsvektoren ähnlich, unterscheiden sich aber in jenen Teilsequenzen, die sie dazu befähigen, in den für die Produktion von Proteinen optimierten Wirtsorganismen zu replizieren und dort das enthaltene Gen zur Expression zu bringen. Bevorzugte Ausführungsformen sind Expressionsvektoren, die selbst die zur Expression notwendigen genetischen Elemente tragen. Die Expression wird beispielsweise von Promotoren beeinflusst, welche die Transkription des Gens regulieren. So kann die Expression durch den natürlichen, ursprünglich vor diesem Gen lokalisierten Promotor erfolgen, aber auch nach gentechnischer Fusion sowohl durch einen auf dem Expressionsvektor bereitgestellten Promotor der Wirtszelle als auch durch einen modifizierten oder einen völlig anderen Promotor eines anderen Organismus.

Bevorzugte Ausführungsformen sind solche Expressionsvektoren, die über Änderungen der Kulturbedingungen oder Zugabe von bestimmten Verbindungen, wie beispielsweise die Zelldichte oder spezielle Faktoren, regulierbar sind. Expressionsvektoren ermöglichen, dass das zugehörige Protein heterolog, also in einem anderen Organismus als dem, aus dem es natürlicherweise gewonnen werden kann, produziert wird. Auch eine homologe Proteingewinnung aus einem das Gen natürlicherweise exprimierenden Wirtsorganismus über einen passenden Vektor liegt innerhalb des Schutzbereichs der vorliegenden Erfindung. Diese kann den Vorteil aufweisen, dass natürliche, mit der Translation in einem Zusammenhang stehende Modifikationsreaktionen an dem entstehenden Protein genauso durchgeführt werden, wie sie auch natürlicherweise ablaufen würden.

Ausführungsformen der vorliegenden Erfindung können auch zellfreie Expressionssysteme sein, bei denen die Proteinbiosynthese *in vitro* nachvollzogen wird. Derartige Expressionssysteme sind im Stand der Technik ebenfalls etabliert.
Die in-vivo-Synthese eines erfindungsgemäßen Enzyms, also die durch lebende Zellen, erfordert den Transfer des zugehörigen Gens in eine Wirtszelle, deren so genannte Transformation. A1s Wirtszellen eignen sich prinzipiell alle Organismen, das heißt Prokaryonten, Eukaryonten oder Archea.

Weitere Gegenstände der vorliegenden Erfindung sind:
Ein Vektor, der einen erfindungsgemäßen Nukleinsäurebereich enthält und der beispielsweise ein Klonierungsvektor oder ein Expressionsvektor sein kann. Außerdem eine Wirtszelle, die eines der erfindungsgemäßen Polypeptide oder Derivate exprimiert oder zu dessen Expression angeregt werden kann, vorzugsweise unter Einsatz eines erfindungsgemäßen Expressionsvektors.
Als Wirtszellen eignen sich prinzipiell alle Organismen, das heißt Prokaryonten, Eukaryonten oder Cyanophyta. Bevorzugt sind solche Wirtszellen, die sich genetisch gut handhaben lassen, was beispielsweise die Transformation mit dem Expressionsvektor und dessen stabile Etablierung angeht, beispielsweise einzellige Pilze oder Bakterien. Zudem zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Häufig müssen aus der Fülle an verschiedenen nach dem Stand der Technik zur Verfügung stehenden Systeme die optimalen Expressionssysteme für den Einzelfall experimentell ermitteln werden. Jedes erfindungsgemäße Protein kann auf diese Weise aus einer Vielzahl von Wirtsorganismen gewonnen werden.

Bevorzugte Ausführungsformen stellen solche Wirtszellen dar, die aufgrund genetischer Regulationselemente, die beispielsweise auf dem Expressionsvektor zur Verfügung gestellt werden, aber auch von vornherein in diesen Zellen vorhanden sein können, in ihrer Aktivität regulierbar sind. Beispielsweise können diese zur Expression angeregt werden durch kontrollierte Zugabe von chemischen Verbindungen wie beispielsweise Methanol, die als Aktivatoren dienen, durch Änderung der Kultivierungsbedingungen oder bei Erreichen einer bestimmten Zelldichte. Dies ermöglicht eine sehr wirtschaftliche Produktion der interessierenden Proteine.
Eine Variante dieses Versuchsprinzips stellen Expressionssysteme dar, bei denen zusätzliche Gene, beispielsweise solche, die auf anderen Vektoren zur Verfügung gestellt werden, die Produktion erfindungsgemäßer Proteine beeinflussen. Hierbei kann es sich um modifizierende Genprodukte handeln oder um solche, die mit dem erfindungsgemäßen Protein gemeinsam aufgereinigt werden sollen, etwa um dessen Funktion zu beeinflussen. Dabei kann es sich beispielsweise um andere Proteine oder Enzyme, um Inhibitoren oder um solche Elemente handeln, die die Wechselwirkung mit verschiedenen Substraten beeinflussen.

Bevorzugte Wirtszellen sind prokaryontische oder bakterielle Zellen. Bakterien zeichnen sich gegenüber Eukaryonten in der Regel durch kürzere Generationszeiten und geringere Ansprüche an die Kultivierungsbedingungen aus. Dadurch können kostengünstige Verfahren zur Gewinnung erfindungsgemäßer Proteine etabliert werden.
Insbesondere bevorzugt sind Wirtszellen, insbesondere Bakterien, die das gebildete Protein oder Derivat ins umgebende Medium sekretieren, so dass die exprimierten erfindungsgemäßen Proteine direkt aufgereinigt werden können.
Bevorzugt ist die heterologe Expression. Grampositive Bakterien, wie beispielsweise Actinomycten oder Bacilli, besitzen keine äußere Membran, so dass sie sekretierte Proteine unmittelbar in das sie umgebende Medium abgeben. Zu den für die heterologe Expression bevorzugten Bakterien gehören somit solche der Gattung Bacillus, insbesondere solche der Species, die unten aufgelistet sind.
Auch gram-negative Bakterien können für die heterologe Expression genutzt werden. Bei ihnen werden eine Vielzahl von Proteinen in den periplasmatischen Raum sekretiert, also in das Kompartiment zwischen den beiden die Zellen einschließenden Membranen. Dies kann für spezielle Anwendungen vorteilhaft sein. Hierzu gehören beispielsweise solche der Gattungen Klebsiella oder Escherichia, bevorzugt der Spezies die unten mit aufgelistet sind. Auch eukaryontische Zellen können sich zur Produktion erfindungsgemäßer Polypeptide eignen. Beispiele dafür sind Hefen wie Saccharomyces oder Kluyveromyces. Dies kann beispielsweise dann besonders vorteilhaft sein, wenn die Proteine im Zusammenhang mit ihrer Synthese spezifische Modifikationen erfahren sollen, die derartige Systeme ermöglichen. Dazu gehören beispielsweise die Bindung niedermolekularer Verbindungen wie Membrananker oder Oligosaccaride.

Besonders bevorzugt für die Produktion erfindungsgemäßer Polypeptide aus transformierten Wirtszellen sind Mikroorganismen, die ausgewählt sind aus der Gruppe, die gebildet wird von *Candida parapsilosis, Saccharomyces cerevisiae, Schizosaccharomyces pombe, Pichia pastoris, Pichia boidinii, Pichia stipitis, Hansenula polymorpha, Kluyveromyces lactis, Schwanniomyces castellii, Yarrowia lipolytica, Escherishia coli, Bacillus subtilis, Bacillus amylolichefaciens, Bacillus stearothermophilus, Bacillus licheniformis, Lactococcus lactis, Streptococcus lactis, Lactobacillus bulgaricus, Aspergillus orizae, Aspergillus niger, Trichoderma reesei, Mucor sp* und *Rhizopus sp..*
Die transformierten Wirtszellen oder auch Transformanten genannt, werden anschließend in an sich bekannter Weise kultiviert und die gebildeten erfindungsgemäßen Polypeptide isoliert.
Die Wirtszellen des erfindungsgemäßen Verfahrens werden in an sich bekannter Weise kultiviert und fermentiert, beispielsweise in diskontinuierlichen oder kontinuierlichen Systemen. Im ersten Fall wird ein geeignetes Nährmedium mit den Organismen beimpft und das Produkt nach einem experimentell zu ermittelnden Zeitraum aus dem Medium geerntet. Kontinuierliche Fermentationen zeichnen sich durch Erreichen eines Fliessgleichgewichts aus, in dem über einen vergleichsweise langen Zeitraum Zellen teilweise absterben aber auch nachwachsen und gleichzeitig Produkt aus dem Medium entnommen werden kann.
Fermentationsverfahren sind an sich aus dem Stand der Technik wohlbekannt und stellen den eigentlichen großtechnischen Produktionsschritt dar; gefolgt von einer geeigneten Aufreinigungsmethode.
Alle Fermentationsverfahren, die auf einem der oben ausgeführten Verfahren zur Herstellung der rekombinanten Proteine beruhen, stellen entsprechend bevorzugte Ausführungsformen dieses Erfindungsgegenstandes dar.
Hierbei müssen die für die eingesetzten Herstellungsverfahren, für die Wirtszellen und/oder die herzustellenden Proteine jeweils optimalen Bedingungen anhand der zuvor optimierten Kulturbedingungen der betreffenden Stämme nach dem Wissen des Fachmanns, beispielsweise hinsichtlich Fermentationsvolumen, Medienzusammensetzung, Sauerstoffversorgung oder Rührergeschwindigkeit experimentell ermittelt werden.
Durch Klonierung und Expression können die erfindungsgemäßen Perhydrolasen in der für den technischen Einsatz erforderlichen Menge zur Verfügung gestellt werden.

Alle bereits oben ausgeführten Elemente können zu Verfahren kombiniert werden, um erfindungsgemäße Polypeptide herzustellen. Diese Verfahren stellen daher einen weiteren Gegenstand der Erfindung dar. Es ist dabei für jedes erfindungsgemäße Protein eine Vielzahl von Kombinationsmöglichkeiten an Verfahrensschritten denkbar. Sie alle verwirklichen die der vorliegenden Erfindung zugrunde liegende Idee, nämlich Vertreter eines über die Perhydrolase Aktivität und gleichzeitig die hohe Homologie zu den in den Sequenzprotokollen angegebenen Sequenzen definierten Proteintyps mithilfe der zugehörigen genetischen Information quantitativ herzustellen. Das optimale Verfahren muss für jeden konkreten Einzelfall experimentell ermittelt werden. Speziell handelt es sich um ein Verfahren zur Herstellung eines Polypeptids oder Derivates gemäß der oben beschriebenen Polypeptide, unter Verwendung einer Nucleinsäure, die für eine Aminosäuresequenz kodiert, welche zu der in SEQ ID NO. 3 angegebenen Aminosäuresequenz wenigstens 80 % Homologie besitzt und/oder unter Verwendung eines Vektors und/oder unter Verwendung einer transformierten Wirtszelle gemäß oben beschriebener Wirtszelle oder unter Verwendung einer Zelle, die dieses natürlicherweise bildet.

Weitere Gegenstände der vorliegenden Erfindung sind Verwendungen der erfindungsgemäßen Polypeptide mit Perhydrolase Aktivität zusammen mit Wasserstoffperoxid, der bei der Verwendung auch *in situ* durch entsprechende Agenzien erzeugt werden kann.

Entscheidend ist die *"in-situ"* Bildung der Persäure durch die enzymatische Reaktion. Dabei ist sehr wichtig, dass es sich um ein selbstkontrollierendes System handelt. Je höher die Temperatur und je höher die Konzentration an Persäure, desto aggressiver wirkt die Persäure. Also muss das System bei höheren Temperaturen (50 - 60° C) weniger Persäure freisetzen. Dies geschieht dadurch, dass das Enzym eine optimale Stabilität bzw. Instabilität zeigt. Mit steigender Temperatur steigt nach physikalischen Gesetzmäßigkeiten die Reaktionsgeschwindigkeit und damit auch die Konzentration der freigesetzten Persäure. Mit zunehmender Konzentration bzw Aktivität der Perhydrolase steigt ebenfalls die Menge der gebildeten Persäure pro Zeiteinheit an. Bei höheren Temperaturen wird aber das Enzym teilweise inaktiviert, Die Inaktivierung verläuft umso schneller, je höher die Temperatur ist. In diesem Perhydrolysesystem nutzt man jetzt die gegenläufigen Effekte der Persäurebildung bei der Temperaturerhöhung. Durch thermische Inaktivierung der Enzyme bei der Temperaturerhöhung wird die Persäurebildung begrenzt, sodass zu hohe, schädigende Persäurekonzentrationen vermieden werden.

Eine lokale Überkonzentration an Persäure auf dem zu behandelnden Gegenstand wird ebenfalls dadurch vermieden, dass die Persäurereaktion nur dann erfolgen kann, wenn sowohl die Perhydrolase gut dispergiert oder gelöst ist und die weiteren Komponenten wie Ester und Wasserstoffperoxid gleichzeitig zur Verfügung stehen. Dies ist dann der Fall, wenn alle Komponenten fein dispergiert oder gelöst sind.

Insgesamt führt das System zu einer limitierten Konzentration an Persäuren. Dadurch werden Schäden durch Bleichreaktion am Gewebe verhindert.
Das Enzym muss ausreichend stabil sein gegen alle weiteren Komponenten in den Wasch- und Reinigungsmittel und zeigt auch ausreichende Stabilität gegen Wasserstoffperoxide.

Der Begriff "Persäure" wird im Sinne der Erfindung mit "Peroxosäure" synonym verwendet.

Das Enzym produziert beispielsweise *in situ* Persäuren aus Fettsäureestern, welche durch die Reaktion des Fettsäureesters mit Wasserstoffperoxid katalysiert wird. Das notwendige Wasserstoffperoxid wird aus Wasserstoffperoxid Quellen oder durch vorhandene Oxidasen gebildet. Die Fettsäureester aus Fettsäuren mit einer Kettenlänge von C7 bis C12 sind besonders geeignet, da sie ein besonders hohes verfügbares Oxidationspotential zeigen und aus nachwachsenden Rohstoffen gewonnen werden können.

Die *in situ* gebildeten Persäuren können in einer weiteren Reaktion mit Olefinen zur Herstellung von Epoxiden verwendet werden.

Eine bevorzugte Ausführungsform der erfindungsgemäßen Verwendung ist die Verwendung in kosmetischen oder pharmazeutischen Mitteln, die Verwendung in Wasch- oder Reinigungsmitteln und/oder die Verwendung zur Bleiche, zur Farbübertragungsinhibierung und zur Desinfektion.

Die erfindungsgemäßen Polypeptide mit Perhydrolase Aktivität können vorteilhaft in Körperpflegemittel, Haarwaschmittel, Haarpflegemittel, Mund-, Zahn- oder Zahnprothesenpflegemittel, Kosmetika, Waschmittel, Reinigungsmittel, Nachspülmittel, Handwaschmittel, Handgeschirrspülmittel, Maschinengeschirrspülmittel, Desinfektionsmittel und Mittel zur bleichenden oder desinfizierenden Behandlung von Filtermedien, Textilien, Pelzen, Papier, Fellen oder Leder, zur desinfizierenden oder bleichenden oder oxidierenden Behandlung von Oberflächen und Lösungen, eingebracht werden. Demgemäß sind die genannten Mittel enthaltend ein erfindungsgemäßes Polypeptid mit Perhydrolase Aktivität gemäß oben beschriebener Polypeptide weitere Gegenstände der Erfindung.

Erfindungsgemäß bevorzugt ist ein Wasch-, oder Bleichmittel, enthaltend ein Bleichsystem, das in der Lage ist, unter Anwendungsbedingungen des Mittels hochaktiven Aktivsauerstoff mittels Wasserstoffperoxid zu erzeugen, und enthaltend gegebenenfalls synthetisches Tensid, organischen und/oder anorganischen Builder sowie sonstige übliche Bleich- oder Waschmittelbestandteile, dadurch gekennzeichnet, dass das Bleichsystem aus einem erfindungsgemäßen Polypeptid mit Perhydrolase Aktivität und einem Substrat für die Perhydrolase (Fett-Vorstufe) sowie einer Wasserstoffperoxid-Quelle oder ein wasserstoffperoxid-erzeugendes System wie Oxidase besteht.

Als **Aktivsauerstoff** (A.O) im Sinne der Erfindung wird der oxidativ wirksame Anteil an Sauerstoff bezeichnet, der im Oxidationsmittel (beispielsweise H₂O₂) enthalten ist. Das Oxidationsmittel H₂O₂ enthält beispielsweise pro Mol H₂O₂ ein Mol Aktivsauerstoff.

Unter **hochaktiven Aktivsauerstoff** wird im Sinne der Erfindung Aktivsauerstoff mit höherem Oxidationspotential als der Aktivsauerstoff der Ausgangsverbindung verstanden.
Beispielsweise wird Wasserstoffperoxid mit einem Aktivsauerstoff zu einer Persäure mit einem Aktivsauerstoff umgesetzt. Die Persäure zeigt jedoch ein höheres Oxidationspotential als Wasserstoffperoxid und damit enthält die Persäure einen "hochaktiven Aktivsauerstoff".

Erfindungsgemäß bevorzugt sind weiterhin die genannten Mittel, die zusätzlich weitere Additive enthalten. Diese weiteren Additive sind bevorzugt ausgewählt aus der Gruppe, die gebildet wird von, Tensiden, Buildermaterial, Buildersubstanzen, Komplexbildner, Weichspüler, Dispergiermittel, Bleichaktivatoren, feste anorganische und/oder organische Säuren beziehungsweise saure Salze, Farbstoffe, Parfums, Pigmente, Vergrauungsinhibitoren, Farbübertragungsinhibitoren und Schauminhibitoren oder Mischungen daraus.

Erfindungsgemäß bevorzugt sind weiterhin die genannten Mittel, die zusätzlich zum Bleichsystem
- 5 Gew.-% bis 70 Gew.-%, insbesondere 10 Gew. -% bis 50 Gew. -% Tensid,
- 10 Gew. -% bis 65 Gew. -%, insbesondere 12 Gew. -% bis 60 Gew.-% wasserlösliches, wasserdispergierbares anorganisches Buildermaterial,
- 1 Gew. -% bis 10 Gew. -%, insbesondere 2 Gew. -% bis 8 Gew.-%, wasserlösliche organische Buildersubstanzen, .
- nicht über 15 Gew. - % feste anorganische und/oder organische Säuren beziehungsweise saure Salze,
- nicht über 5 Gew. -% Komplexbildner für Schwermetalle,
- nicht über 5 Gew. -% Vergrauungsinhibitor,
- nicht über 5 Gew.-% Farbübertragungsinhibitor und
- nicht über 5 Gew. -% Schauminhibitor enthalten.

Das Bleichsystem ist dadurch charakterisiert, dass es auf einem Aktivsauerstoff-Träger aufbaut. Beispielsweise enthält Natrium-Perborat ca. 10% Aktivsauerstoff (A.O), reines Wasserstoffperoxid enthält 47% Aktivsauerstoff.
Das Bleichsystem kann 5 - 95 % Gewichtsprozent eines Aktivsauerstoffträgers enthalten, bevorzugt 10 - 60 %. Dies entspricht einem bevorzugten Aktivsauerstoffgehalt von 1- 6 % A.O in dem Mittel.
Das Bleichsystem enthält außerdem ein unten beschriebenes Substrat.

Erfindungsgemäß enthalten die genannten Mittel 0,00001 - 3 Gew-% der erfindungsgemäßen Polypeptide nach oben genannter Beschreibung und Definition berechnet als aktives Substanz bezogen auf 100 % reinen aktiven Katalysator bezogen auf die Gesamtmenge des Mittels inklusive Wasser und gegebenenfalls genannter Additive addiert zu 100 %.
Die Perhydrolase wird üblicherweise als konfektioniertes Enzym eingesetzt, wobei die aktive Substanz üblicherweise zwischen 0,05 bis 10,0 % des konfektionierten Enzymprodukts betragen kann, ohne dass dies für die Erfindung als Einschränkung zu betrachten ist. Für die Erfindung kann ebenfalls die Aktivsubstanz direkt verwendet werden.

Bevorzugt ist eine Menge von 0,00001 - 2 Gew.-% aktiver Substanz und insbesondere bevorzugt eine Menge von 0,00005 bis 0,3 Gew.-% aktiver Substanz. Die Perhydrolase in konfektionierter Form wird bevorzugt in Mengen von 0,01 bis 50 Gew.-% und besonders bevorzugt von 0,1 bis 10 Gew.-% eingesetzt.

Das Substrat für die Perhydrolase (Fett-Vorstufe) wird vorzugsweise gebildet von einer Verbindung mit der allgemeinen Formel (I)

R¹Oₓ[(R²)ₘ(R³)ₙ]ₚ (I)

(i) wobei R1 ausgewählt ist aus der Gruppe, die gebildet wird von H, substituierte oder unsubstituierte Alkyl-, Heteroalkyl-, Alkenyl-, Alkynyl-, Aryl-, Alkylaryl-, Alkylheteroaryl- oder Heteroarylgruppen, primäre-, sekundäre-, tertiäre-, oder quatäre Aminreste,
(ii) wobei R2 eine Alkoxylgruppe darstellt,
(iii) wobei R3 einen esterbildenden Rest darstellt mit der Formel (II) R⁴CO- worin R⁴ H, Alkyl-, Alkenyl-, Alkynyl-, Aryl-, Alkylaryl-, Alkylheteroaryl- oder Heteroarylgruppen darstellt,
(iv) x ist 1 wenn R1 H ist und wenn R1 nicht H ist, entspricht x kleiner oder gleich der Anzahl an C-Atomen aus R1,
(v) p ist kleiner oder gleich x,
(vi) m ist eine ganze Zahl zwischen 0 und 50 und
(vii) n ist eine ganze Zahl und wenigstens 1.

Das Substrat ist vorzugsweise ausgewählt aus Fettsäureestern mit einem Fettsäurerest aus 6 bis 14 C-Atomen oder aus Fettsäureestern aus Polycarbonsäuren, insbesondere C12-Dicarbonsäure oder speziell ausgewählt unter Methyl-, Ethyl- oder Glycerinestern von Butter-, Capron- Octansäure, oder Decansäure bevorzugt Capronsäuremethylester, Octansäuremethylester, Trioctanoin und besonders bevorzugt sind biologisch gut abbaubare Ester: Triglyceride, Partialglyceride, Methyl- und Ethylester mit Kettenlängen von C7 - C12. Die Menge des im erfindungsgemäßen Waschmittel enthaltenen Substrats für die Perhydrolase richtet sich nach der zum Erzielen des gewünschten Bleichergebnisses erforderlichen Percarbonsäuremenge und kann durch den Fachmann in gewünschter Weise eingestellt werden.

Die **Oxidasen** sind vorzugsweise ausgewählt aus der Gruppe, die gebildet wird von Aldose oxidase, Galactose oxidase, Cellobiose oxidase, Pyranose oxidase, Verbose oxidase, Hexose oxidase, Glucose oxidase und Mischungen daraus.

Die Mittel enthalten bevorzugt **Wasserstoffperoxidquelle** ausgewählt aus der Gruppe, die gebildet wird aus Alkalimetallpercarbonate, -perphosphate, -persulfate, organische Persäuren und Harnstoff-Wasserstoffperoxid oder Mischungen daraus und spezielle ausgewählt aus Natriumpercarbonat, Natriumperborat oder Kaliummonopersulfat-Tripelsalz, Salzmischungen von Percarbonat / Kaliumpersulfat im Verhältnis 1 zu 3 (pH 5,5); Percarbonat / Kaliumpersulfat im Verhältnis 1 zu 1 (pH 7,5); Percarbonat / Kaliumpersulfat im Verhältnis 3 zu 1 (>pH 9) und besonders bevorzugt H₂O₂.

Ein erfindungsgemäßes Polypeptid mit Perhydrolase Aktivität wird vorzugsweise in solchen Mengen eingesetzt, dass das gesamte Mittel eine Perhydrolase-Aktivität von 0,05 bis 5 ppmAO/µg vorzugsweise 0,2 bis 1,2 ppmAO/µg aufweist. Mittel mit Perhydrolase-Aktivitäten in den genannten Bereichen weisen eine für übliche europäische maschinelle Waschverfahren ausreichend rasche Percarbonsäurefreisetzung auf, wohingegen die Steigerung der enthaltenen Perhydrolasemenge auf höhere Aktivitäten in der Regel keine entsprechend hohe Steigerung der Bleichleistung ergibt.

Ein erfindungsgemäßes Polypeptid mit Perhydrolase-Aktivität wird vorzugsweise in solchen Mengen eingesetzt, dass eine ausreichend rasche Percarbonsäurefreisetzung bei der Anwendung erfolgt. Angestrebt ist außerdem eine kontinuierliche Nachlieferung der Percarbonsäure über den Anwendungszeitraum zu ausreichend hohen Wirkkonzentrationen der gebildeten Percarbonsäure. Dabei wird in der Regel die Bildung von 0,1 bis maximal 100 ppm Aktivsauerstoff der Percarbonsäure einer verdünnten Lösung über den Anwendungszeitraum angestrebt. Die Steigerung der enthaltenen Perhydrolasemenge auf höhere Aktivitäten führt in der Regel nicht zu höheren Bleichleistungen. Die Perhydrolase wird vorzugsweise in solchen Mengen eingesetzt, dass das Gew.-Verhältnis Enzym (bezogen auf aktive Substanz) zu theoretisch möglicher Aktivsauerstoffbildung der Percarbonsäure im Gew.-Verhältnis von 100: 1 bis 1:500, bevorzugt 10:1 bis 1:100 beträgt.

Wasch -und Reinigungsmittel beinhalten jede Form von Waschmittel für Textilien beispielsweise in Pulverform, als Gel oder als Flüssigwaschmittel. Es werden des weiteren alle möglichen Reinigungsmittel verstanden, die zur Bleiche oder Desinfizierung von Textilien wie Kleidung, Bettwäsche, Gardinen, und Dekorationsmaterialien sowie zur Desinfizierung von Oberflächen verwendet werden können.
Die kosmetischen und/oder pharmazeutischen Mittel können beispielsweise in Form von Schaumbäder, Duschbäder, Cremes, Gele, Lotionen, alkoholische und wässrig/alkoholische Lösungen, Emulsionen, Wachs/ Fett-Massen, Stiftpräparate, Puder oder Salben vorliegen und die erfindungsgemäßen Polypeptide mit Perhydrolase Aktivität umfassen. Diese kosmetischen und/oder pharmazeutischen Mittel können als Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deodorantien, Antitranspirantien, Antischuppenmittel, Filmbildner, Quellmittel, UV-Licht-schutzfaktoren, Antioxidantien, Hydrotrope, Konservierungsmittel, Insektenrepellentien, Selbstbräuner, Solubilisatoren, Parfümöle, Farbstoffe und dergleichen enthalten.

Die genannten Mittel sind erfindungsgemäß bevorzugt dadurch gekennzeichnet, dass sie als Tab, Granulat oder als rieselfähiges Pulver mit einem Schüttgewicht von 300 g/l bis 1200 g/l, insbesondere 500 g/l bis 900 g/l, vorliegen.
Die Mittel können des Weiteren bevorzugt in Form eines pastenförmigen oder flüssigen Waschmittels vorliegen.

Die erfindungsgemäßen Mittel können des weiteren neben einzelnen oder allen genannten Bedingungen zusätzlich Enzymstabilisatoren und/oder weitere Enzyme insbesondere Proteasen, Amylasen, Cellulasen, Hemicellulasen, Cutinasen, Pectinasen, Mannanasen, Endoglycosidasen, Lysozyme, Zellwandabbauende Enzyme, weitere Oxidoreduktasen und/oder Lipasen enthalten.

Die Verwendung der genannten Mittel mit einzelnen, mehreren oder allen genannten weiteren Ausführungsformen in kosmetischen oder pharmazeutischen Mitteln, in Wasch- oder Reinigungsmitteln und/oder zur Bleiche, zur Farbübertragungsinhibierung und zur Desinfektion stellen weitere Ausführungsformen der Erfindung dar.

### Beispiele

### Beispiel 1: Bestimmung der Enzymaktivität

Die hydrolytische Aktivität (standard units) wurde untersucht durch Mischung von 1 ml eines Enzymextraktes in 50 mM Phosphatpuffer pH 6,5 mit 10 µmol Decansäuremethylester (in 50 µl Aceton). Die Reaktion wurde unter Rühren 15 min. bei 30 °C in verschlossenen Wheaton Gefäßen durchgeführt und gestoppt durch die Zugabe von 0.95 ml Ethanol/H₂SO₄ (100/0,8). 50 µl eines internen Standards bestehend aus 1,5 µmol Laurinsäuremethylester (C12-Methylester) und 1,5 µmol Laurinsäure wurden der Lösung zugegeben.
Als Vergleich wurde hergestellt durch Mischung von Ethanol//H₂SO₄ zur Lösung ohne Enzym.
Die Fette wurden extrahiert mit 1 ml Hexan und analysiert durch GC nach Silylierung. Dafür wurden 25 µl MSTFA ((N-methyl-N-trimethylsilyl)trifluoroacetamid) und 25 µl Pyriding zu 200 µl Hexan-Extrakt gegeben. Die Mischung wurde für 20 min bei 50°C inkubiert bevor sie in den GC injiziert wurde. Der GC (HP 5890 II mit Autosampler) ist ausgestattet mit einer J&W DB1 Säule (30 m, 0,32 mm Durchmesser, 0,1 Säule µm Film Dicke), ein Split Injector und einer FID und Waters Millenium32 acquisition und integration software. Vector Gas war Helium mit 28 ml/min. Der Decansäuremethylester und die Decansäure wurden untersucht mit Laurinsäuremethylester und Laurinsäure als internen Standard.

Ergebnisse: Die eingesetzte Perhydrolase zeigte eine Aktivität von 9,7 µmol freigesetzter Decansäure pro Minute pro mg Perhydrolase bei 30° C.

### Beispiel 2: Persäure Bestimmung

Der Gehalt an Persäure wurde bestimmt unter Verwendung von ABTS (2-2'-azino-bis(-ethylbenzothiazolin-6-sulfonsäure) als Indikator. Das Reaktionsmedium bestand aus 100 µl zu untersuchende Lösung, 200 µl Essigsäure 1 M, 50 µl KI 100 mg/l, 550 µl Wasser und 100 µl ABTS 1 g/l. Die Reaktion wurde gestartet durch das Hinzufügen von ABTS zur zu untersuchenden Lösung. Die vorhandene, zu bestimmende Persäure oxidiert ABTS zum Radikal, welches dann in blauer Farbe erscheint. Die Absorbtion wurde nach 10 min Inkubation bei Raumtemperatur bei einer Wellenlänge von 405 nm bestimmt. Die Standard Kurve wurde erhalten durch unterschiedliche Konzentration an zu untersuchenden Lösungen.
Eine Kalibrierung erfolgte über die photometrische Absorptionsmessung des ABTS-Reaktionsprodukts durch definierte Peressigsäurekonzentrationen.
1 µmol/ml Peressigsäure entspricht unter diesen Testbedingungen einer Extinktion von 0,75.

### Beispiel 3: Perhydrolyse Aktivität und Selektivität des Enzyms

Das Enzym-Reaktionsmedium enthielt in einem Volumen von 1 ml: 10 µmol Decansäuremethylester 250, 500 und 1000 ppm H₂O₂, 2 µg Enzym (Perhydrolase mit einer Aminosäuresequenz von mindestens 99,5 % Identität mit SEQ ID NO 3), und Phosphat Puffer pH 6,5, 50 mM. Die Reaktion wurde bei 30 °C und 45 °C durchgeführt und gestartet durch die Zugabe des Enzyms (Perhydrolase mit einer Aminosäuresequenz von mindestens 99,5 % Identität mit SEQ ID NO 3). Gestoppt wurde die Reaktion nach 15 min. durch die Zugabe von 1 ml Ethanol.
Ein Vergleich wurde hergestellt, indem das Enzym durch Wasser ersetzt wurde. Es wurden die gleichen Bedingungen eingehalten. Auch bei dem Vergleich wurde Ethanol zugegeben um einen Nullpunkt bestimmen zu können.
Da die Perhydrolase sowohl Säure als auch Persäure bildet, wird ein Gesamtsäuregehalt und ein Persäuregehalt bestimmt. Aus beiden Messungen kann dann die Selektivität der Reaktion bestimmt werden.

**Tabelle 1: Perhydrolase Aktivität**

| Temperatur | Enzymlösung | Substrat | Acid + Peracid | Peracid | H₂O₂ | Gesamt-Hydrolyse-Aktivität | Fettsäure-Hydrolyse-Aktivität | Perhydrolyse -Aktivität |
|---|---|---|---|---|---|---|---|---|
| | µg Protein / ml | C10Me | nach 15 min | nach 15 min | | | | |
| | | µmol | µmol/ml | µmol/ml | (ppm) | µmol/min/mg | µmol/min/mg | µmol/min/mg |
| 30°C | 2 | 10 | 0,29 | 0,04 | 250 | 9,7 | 8,3 | 1,3 |
| | | | 0,29 | 0,213 | 500 | 9,7 | 2,6 | 7,1 |
| | | | 0,29 | 0,2 | 1000 | 9,7 | 3,0 | 6,7 |
| | | | | | | | | |
| 45°C | 2 | 10 | 0,58 | 0,02 | 250 | 19,3 | 18,7 | 0,7 |
| | | | 0,68 | 0,22 | 500 | 22,7 | 15,3 | 7,3 |
| | | | 0,57 | 0,18 | 1000 | 19,0 | 13,0 | 6,0 |

**Tabelle 2: Selektivität des Enzyms bei 30°C und 45 °C**

| H₂O₂ (ppm) | Hydrolyse-Aktivität | Fettsäure-Hydrolyse-Aktivität | Perhydrolyse-Aktivität | Selektivität Persäure / Säure |
|---|---|---|---|---|
| | µmol/min/mg | µmol/min/mg | µmol/min/mg | |
| **30 °C** | | | | |
| 250 | 9,67 | 8,33 | 1,33 | 0,16 |
| 500 | 9,67 | 2,57 | 7,10 | 2,77 |
| 1000 | 9,67 | 3,00 | 6,67 | 2,22 |
| **45 °C** | | | | |
| 250 | 19,33 | 18,67 | 0,67 | 0,04 |
| 500 | 22,67 | 15,33 | 7,33 | 0,48 |
| 1000 | 19,00 | 13,00 | 6,00 | 0,46 |

Das Beispiel zeigt, dass die Selektivität bei 30°C und 500 ppm H₂O₂ größer als 2,5 erreicht werden.
Bei höherem Temepraturen, bei denen zu hohe Konzentrationen an Persäure schädigend für das zu behandelnde Material werden kann, sinkt die Selektivität auch bei höheren H₂O₂-Konzentrationen vorteilhaft unter 0,5. Damit ist das Ziel eines selbstkontrollierenden Systems erreicht.

### Beispiel 4 : Enzymstabilität- und Aktivitätstest in Gegenwart von Wasserstoffperoxid

Für Stabilitätstest wurden 90 µg Enzym (Perhydrolase mit einer Aminosäuresequenz von mindestens 99,5 % Identität mit SEQ ID NO 3) bei 30 °C oder 45 °C in geschlossenen Gefäßen unter Rühren 0, 15, 30 und 60 Minuten inkubiert in Gegenwart unterschiedlicher H₂O₂ Konzentrationen. Anschließend wurde mit diesen Enzymlösungen ein Enzymaktivitätstest wie unter Beispiel 1 durchgeführt.

**Tabelle 3: Ergebnis der Aktivitätsbestimmung (µmol/min/mg) bei 30 °C nach Vorinkubation ohne H₂O₂ und mit verschiedenen H₂O₂-Konzentrationen.**

| | | | | |
|---|---|---|---|---|
| Die Auswertung erfolgt dadurch, dass die Enzymaktivität bei 0 Minuten Inkubation gleich 100% gesetzt wird und die Stabilität anhand der Restaktivitäten beurteilt wird | | | | |

| H₂O₂ in % | 0 min | 15 min | 30 min | 60 min |
|---|---|---|---|---|
| 0 | 4,7 | 4,5 | 4,1 | 4,1 |
| 2,25 | 5,2 | 3,7 | 4,1 | 4,6 |
| 11.25 | 7,0 | 3,8 | 4,4 | 5,7 |
| 16,9 | 11,8 | 3,4 | 3,9 | 5,0 |
| 22,5 | 8,2 | 3,4 | 2,9 | 2,1 |

**Tabelle 4: Restaktivität bei 30°C Vorinkubation mit Wasserstoffperoxid**

| H₂O₂ in % | Restaktivität nach Vorinkubation bei 30°C in Minuten | | | |
|---|---|---|---|---|
| | 0 | 15 | 30 | 60 |
| 0,00 | 100% | 96% | 87% | 87% |
| 2,25 | 100% | 71% | 79% | 88% |
| 11,25 | 100% | 54% | 63% | 81% |
| 16,90 | 100% | 29% | 33% | 42% |
| 22,50 | 100% | 41% | 35% | 26% |

**Tabelle 5: Ergebnis der Aktivitätsbestimmung (µmol/min/mg) bei 45 °C**

| H₂O₂ in % | 0 min | 15 min | 30 min | 60 min |
|---|---|---|---|---|
| 0 | 8,1 | 6,9 | 6,2 | 6,1 |
| 2,25 | 7,2 | 7,1 | 6,0 | 5,2 |
| 11.25 | 6,8 | 7,0 | 5,3 | 4,1 |
| 16,9 | 6,8 | 2,2 | 1,7 | 0,3 |
| 22,5 | 2,3 | 0,1 | 0 | 0 |

**Tabelle 6: Restaktivität bei 45 °C Vorinkubation mit Wasserstoffperoxid**

| H₂O₂ in % | Restaktivität nach Vorinkubation bei 30°C in Minuten | | | |
|---|---|---|---|---|
| | 0 | 15 | 30 | 60 |
| 0,00 | 100% | 85% | 77% | 75% |
| 2,25 | 100% | 99% | 83% | 72% |
| 11,25 | 100% | 103% | 78% | 60% |
| 16,90 | 100% | 32% | 25% | 4% |
| 22,50 | 100% | 4% | 0% | 0% |

Das erfindungsgemäße Enzym zeigt eine gute Stabilität bei einer H₂O₂- Konzentrationen bis zu 11 %, insbesondere bei 30 °C. Bei höheren Reaktionstemperaturen nimmt die Stabilität bei Konzentration größer 11 % schneller ab.

### Beispiel 5: Bleichversuche

Es wurden 1,9 mg Decansäuremethylester als PIT-Emulsion (Phase-inverse-temperatur) bei pH = 6,5 mit 550 ppm Wasserstoffperoxid und 9 µg Perhydrolase/ml bei 45 ° C zur Reaktion gebracht.

Als Vergleich diente die gleiche Lösung ohne Perhydrolase.
Diese Mischungen wurden 2 min, 15 min, oder 60 min auf Textilproben, welche mit Rotwein oder mit gelbem Farbstift beschmutzt waren aufgetragen.
Bereits nach 15 min konnte ein Unterschied in der Bleichwirkung sowohl bei Rotwein als auch bei dem Farbstift beobachtet werden. Nach 60 min war der Bleicherfolg mit Enzym erheblich größer als ohne Enzym. Mit Enzym konnte die Verschmutzung fast vollständig entfernt werden.

### Annex zu den Anmeldungsunterlagen - nachgereichtes Sequenzprotokoll

## Patentansprüche

1. Polypeptide mit Perhydrolase Aktivität mit einer Aminosäuresequenz, welche zu der in SEQ ID NO 3 angegebenen Aminosäuresequenz wenigstens 80 % Homologie besitzt, mit Ausnahme der SEQ ID NO 3.

2. Polypeptide mit Perhydrolase Aktivität mit einer Aminosäuresequenz, welche zu der in SEQ ID NO 3 angegebenen Aminosäuresequenz wenigstens 65% Identität besitzt, mit Ausnahme der SEQ ID NO 3.

3. Polypeptide mit Perhydrolase Aktivität mit einer Aminosäuresequenz von mindestens 200 Aminosäuren, welche nach Sequenzvergleich durch alignment mit der SEQ ID NO 3 in den überlappenden Bereichen der Aminosäuresequenzen wenigstens 80 % Homologie zur SEQ ID NO 3 aufweisen, mit Ausnahme der SEQ ID NO 3.

4. Polypeptide mit Perhydrolase Aktivität mit einer Aminosäuresequenz von mindestens 200 Aminosäuren, welche nach Sequenzvergleich durch alignment mit der SEQ ID NO 3 in den überlappenden Bereichen der Aminosäuresequenzen wenigstens 65 % Identität zur SEQ ID NO 3 aufweisen, mit Ausnahme der SEQ ID NO 3.

5. Polypeptide mit Perhydrolase Aktivität mit einer Aminosäuresequenz, welche zu der in SEQ ID NO 3 angegebenen Aminosäuresequenz in den Positionen äquivalent zu Positionen 170 bis 380 wenigstens 80 % Homologie besitzt mit Ausnahme der SEQ ID NO 3.

6. Polypeptide mit Perhydrolase Aktivität mit einer Aminosäuresequenz, welche zu der in SEQ ID NO 3 angegebenen Aminosäuresequenz in den Positionen äquivalent zu Positionen 170 bis 380 wenigstens 65 % Identität besitzt, mit Ausnahme der SEQ ID NO 3.

7. Polypeptide mit Perhydrolase Aktivität nach einem der Ansprüche 1 bis 6, **dadurch gekennzeichnet, dass** es sich um strukturelle Homologe handelt, bei denen das Aktive Zentrum identisch ist zu wenigstens einer Aminosäure die äquivalent ist zu Positionen in der Aminosäuresequenz der SEQ ID NO 3 die ausgewählt sind aus der Gruppe die gebildet wird aus S 180, D332 und H365 der SEQ ID NO 3.

8. Polypeptide mit Perhydrolase Aktivität enthaltend wenigstens ein Motif, welches wenigstens 50 % Homologie zeigt zu einem Motif ausgewählt aus der Gruppe, die gebildet wird aus
SEQ ID NO 4: GYSGGxxAxxWAxxxxxxYAPE,
SEQ ID NO 5: GYSGGxxAxxWAxxxxxxYAPD,
SEQ ID NO 6: GFSGGxxAxxWAxxxxxxYAPE,
SEQ ID NO 7: GFSGGxxAxxWAxxxxxxYAPD,
SEQ ID NO 8: GYSGGxxAxxWAxxxxxxYA und
SEQ ID NO 9: GFSGGxxAxxWAxxxxxxYA.

9. Polypeptide mit Perhydrolase Aktivität enthaltend wenigstens ein Motif, welches wenigstens 70 % Identität zeigt zu einem Motif ausgewählt aus der Gruppe, die gebildet wird aus
SEQ ID NO 4: GYSGGxxAxxWAxxxxxxYAPE,
SEQ ID NO 5: GYSGGxxAxxWAxxxxxxYAPD,
SEQ ID NO 6: GFSGGxxAxxWAxxxxxxYAPE,
SEQ ID NO 7: GFSGGxxAxxWAxxxxxxYAPD,
SEQ ID NO 8: GYSGGxxAxxWAxxxxxxYA und
SEQ ID NO 9: GFSGGxxAxxWAxxxxxxYA.

10. Für ein Polypeptid mit Perhydrolase Aktivität kodierende Nucleinsäuren, deren Nucleotidsequenzen zu der in SEQ ID NO. 1 angegebenen Nucleotidsequenz wenigstens 80 % Homologie besitzt, mit Ausnahme von SEQ ID NO. 1.

11. Für ein Polypeptid mit Perhydrolase Aktivität kodierende Nucleinsäuren, deren Nucleotidsequenzen zu der in SEQ ID NO. 1 angegebenen Nucleotidsequenz zu wenigstens 65 % identisch sind, mit Ausnahme von SEQ ID NO. 1.

12. Vektor, insbesondere Klonierungsvektor oder Expressionsvektor, der eine in den Ansprüchen 10 bis 11 bezeichnete Nukleinsäure enthält.

13. Transformierte Wirtszelle, insbesondere Wirtszellen aus Mikroorganismen, die eines der in den Ansprüchen 1 bis 9 bezeichneten Polypeptide oder Derivate exprimiert oder zu dessen Expression angeregt werden kann.

14. Körperpflegemittel, Haarwaschmittel, Haarpflegemittel, Mund-, Zahn- oder Zahnprothesenpflegemittel, Kosmetika, Waschmittel, Reinigungsmittel, Nachspülmittel, Handwaschmittel, Handgeschirrspülmittel, Maschinengeschirrspülmittel, Desinfektionsmittel und Mittel zur bleichenden oder desinfizierenden Behandlung von Filtermedien, Textilien, Pelzen, Papier, Fellen oder Leder zur desinfizierenden oder bleichenden oder oxidierenden Behandlung von Oberflächen und Lösungen, enthaltend ein Polypeptid mit Perhydrolase Aktivität gemäß einem der Ansprüche 1 bis 9.

15. Wasch-, oder Bleichmittel, enthaltend ein Bleichsystem, das in der Lage ist, unter Anwendungsbedingungen des Mittels Percarbonsäure zu erzeugen, und gegebenenfalls synthetisches Tensid, organischen und/oder anorganischen Builder sowie sonstige übliche Bleich- oder Waschmittelbestandteile, **dadurch gekennzeichnet, dass** das Bleichsystem ein Polypeptid mit Perhydrolase Aktivität gemäß einem der Ansprüche 1 bis 9, eine Wasserstoffperoxid-Quelle oder ein wasserstoffperoxid-erzeugendes System wie Oxidase und ein Substrat für die Perhydrolase (Fett-Vortsufe) enthält.
